# EUROPEAN PATENT APPLICATION

(11) **EP 3 673 918 A1**
(43) Date of publication of application: **01.07.2020**
(21) Application number: 18849166.6
(22) Date of filing: 22.08.2018
(51) Int. Cl.: A61K 39/395, A61K 9/08, A61K 9/19, A61K 31/4745, A61K 47/10, A61K 47/22, A61K 47/26, A61K 47/68, A61P 35/00, A61P 35/02, C07K 16/00, C07K 19/00

(54) **ANTIBODY-DRUG CONJUGATE PREPARATION AND LYOPHILIZATION FOR SAME**

(30) Priority: 23.08.2017 JP 2017160071
(71) Applicant: Daiichi Sankyo Company, Limited, Tokyo 103-8426 (JP)
(72) Inventor: KAMII, Tetsuo, Tokyo 103-8426 (JP); NISHIMOTO, Norihiro, Tokyo 103-8426 (JP)
(74) Representative: Fairbairn, Angus Chisholm
(86) International application number: PCT/JP2018/030882
(87) International publication number: WO 2019/039483

(57) **Abstract**

A pharmaceutical composition comprising (i) an antibody-drug conjugate in which a drug-linker represented by the following formula (wherein A represents a connecting position to an antibody) is conjugated to the antibody via a thioether bond, (ii) a histidine buffer, (iii) sucrose or trehalose, and (iv) a surfactant; and a method of lyophilizing the pharmaceutical composition.

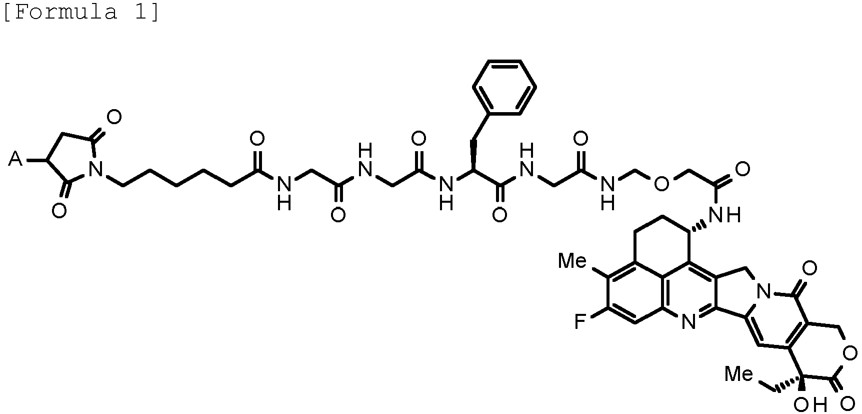

## Description

### Technical Field

The present invention relates to a specific antibody-drug conjugate formulation and a method of lyophilizing the formulation.

### Background Art

An antibody-drug conjugate (ADC) having a drug with cytotoxicity conjugated to an antibody, whose antigen is expressed on the surface of cancer cells and which also binds to an antigen capable of cellular internalization, and therefore can deliver the drug selectively to cancer cells, is thus expected to cause accumulation of the drug within cancer cells and to kill the cancer cells (Non-Patent Literatures 1 to 5).

As one such antibody-drug conjugate, an antibody-drug conjugate comprising an antibody and exatecan, which is a topoisomerase I inhibitor, as its components is known (Patent Literatures 1 to 8, and Non-Patent Literatures 6, 7). Since these antibody-drug conjugates exert a particularly superior antitumor effect and have safety, they are currently under clinical studies.

As formulations of such antibody-drug conjugates, a formulation of an antibody-drug conjugate comprising maytansinoid as a component (Patent Literatures 9 to 12), a formulation of an antibody-drug conjugate comprising monomethyl auristatin E as a component (Patent Literatures 13 to 15), a formulation of an antibody-drug conjugate comprising SN-38 as a component (Patent Literature 16) and the like are known.

### Citation List

### Patent Literatures

Patent Literature 1: International Publication No. WO 2014/057687
Patent Literature 2: International Publication No. WO 2014/061277
Patent Literature 3: International Publication No. WO 2015/098099
Patent Literature 4: International Publication No. WO 2015/115091
Patent Literature 5: International Publication No. WO 2015/146132
Patent Literature 6: International Publication No. WO 2015/155976
Patent Literature 7: International Publication No. WO 2015/155998
Patent Literature 8: International Publication No. WO 2018/135501
Patent Literature 9: International Publication No. WO 2004/004639
Patent Literature 10: International Publication No. WO 2004/110498
Patent Literature 11: International Publication No. WO 2007/019232
Patent Literature 12: International Publication No. WO 2015/059147
Patent Literature 13: International Publication No. WO 2010/081004
Patent Literature 14: International Publication No. WO 2014/143765
Patent Literature 15: International Publication No. WO 2015/157286
Patent Literature 16: International Publication No. WO 2014/092804

### Non-Patent Literatures

Non-Patent Literature 1: Ducry, L., et al., Bioconjugate Chem. (2010) 21, 5-13.
Non-Patent Literature 2: Alley, S. C, et al., Current Opinion in Chemical Biology (2010) 14, 529-537.
Non-Patent Literature 3: Damle N. K Expert Opin. Biol. Ther. (2004) 4, 1445-1452.
Non-Patent Literature 4: Senter P. D, et al., Nature Biotechnology (2012) 30, 631-637.
Non-Patent Literature 5: Howard A. et al, J Clin Oncol 29: 398-405.
Non-Patent Literature 6: Ogitani Y. et al., Clinical Cancer Research (2016) 22 (20), 5097-5108.
Non-Patent Literature 7: Ogitani Y. et al., Cancer Science (2016) 107, 1039-1046.

### Summary of Invention

### Technical Problem

In antibody formulations, the formation of aggregates and the generation of decomposition products cause medically undesirable effects, for example, they become a factor of immunogenicity or venous disorders for patients receiving the formulations. Thus, when formulating antibodies, it is required to suppress the formation of aggregates and the generation of decomposition products, and in view of this, various pharmaceutical compositions (e.g., in the forms of an aqueous injection and a lyophilized injection) have been studied.

However, when formulating antibody-drug conjugates, more complicated studies are necessary, because it is necessary to consider not only the specific properties of the antibody part, but also the specific properties of the drug-linker part.

Furthermore, when preparing a lyophilized injection from an aqueous solution containing sucrose or trehalose, there are problems such as (1) the primary drying process is long, and (2) shrinkage tends to occur in the lyophilized cake.

Thus, a main object of the present invention is to provide, for a specific antibody-drug conjugate, a pharmaceutical composition (especially in the forms of an aqueous injection and a lyophilized injection) wherein the formation of aggregates and the generation of decomposition products is suppressed, and an efficient method of lyophilizing an aqueous solution to a lyophilized injection.

### Solution to Problem

The present inventors have found, for a specific antibody-drug conjugate, a pharmaceutical composition (especially in the forms of an aqueous injection and a lyophilized injection) in which the formation of aggregates and the generation of decomposition products is suppressed, and also found an efficient method of lyophilizing an aqueous solution to a lyophilized injection.

Specifically, the present invention relates to the following.
[1] A pharmaceutical composition comprising
   (i) an antibody-drug conjugate,
   (ii) a histidine buffer,
   (iii) sucrose or trehalose, and
   (iv) a surfactant,
   wherein the antibody-drug conjugate is an antibody-drug conjugate in which a drug-linker represented by the following formula: 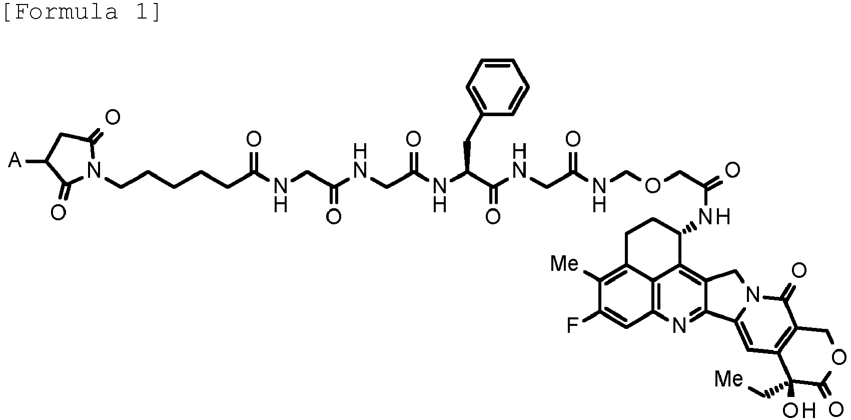 wherein A represents a connecting position to an antibody, is conjugated to the antibody via a thioether bond.
[2] The pharmaceutical composition according to [1], wherein the surfactant is polysorbate 80 or polysorbate 20.
[3] The pharmaceutical composition according to [1] or [2] comprising,
   (i) per 20 mg of the antibody-drug conjugate,
   (ii) 3 to 80 mmol of the histidine buffer,
   (iii) 24 to 320 mg of sucrose or trehalose, and
   (iv) 0.05 to 1.6 mg of polysorbate 80 or polysorbate 20.
[4] The pharmaceutical composition according to any one of [1] to [3] comprising,
   (i) per 20 mg of the antibody-drug conjugate,
   (ii) 10 to 40 mmol of the histidine buffer,
   (iii) 90 mg of sucrose or 100 mg of trehalose hydrate, and
   (iv) 0.2 to 0.4 mg of polysorbate 80 or polysorbate 20.
[5] The pharmaceutical composition according to [1] or [2] comprising
   (i) the antibody-drug conjugate,
   (ii) the histidine buffer,
   (iii) sucrose, and
   (iv) polysorbate 80 or polysorbate 20.
[6] The pharmaceutical composition according to [1], [2] or [5] comprising,
   (i) per 20 mg of the antibody-drug conjugate,
   (ii) 3 to 80 mmol of the histidine buffer,
   (iii) 24 to 320 mg of sucrose, and
   (iv) 0.05 to 1.6 mg of polysorbate 80 or polysorbate 20.
[7] The pharmaceutical composition according to [1], [2], [5] or [6] comprising,
   (i) per 20 mg of the antibody-drug conjugate,
   (ii) 10 to 40 mmol of the histidine buffer,
   (iii) 90 mg of sucrose, and
   (iv) 0.2 to 0.4 mg of polysorbate 80 or polysorbate 20.
[8] The pharmaceutical composition according to any one of [1] to [7] comprising,
   (i) per 20 mg of the antibody-drug conjugate,
   (ii) 10 or 25 mmol of the histidine buffer,
   (iii) 90 mg of sucrose, and
   (iv) 0.2 or 0.3 mg of polysorbate 80 or polysorbate 20.
[9] The pharmaceutical composition according to any one of [1] to [8], wherein the pH of the composition when the antibody-drug conjugate is dissolved in water at a concentration of 20 mg/mL is 4.0 to 7.0.
[10] The pharmaceutical composition according to any one of [1] to [9], wherein the average number of units of the drug-linker conjugated per antibody molecule in the antibody-drug conjugate is in the range of from 2 to 8.
[11] The pharmaceutical composition according to any one of [1] to [10], wherein the antibody in the antibody-drug conjugate is an anti-HER2 antibody, an anti-HER3 antibody, an anti-TROP2 antibody, an anti-B7-H3 antibody, or an anti-GPR20 antibody.
[12] The pharmaceutical composition according to [11], wherein the antibody in the antibody-drug conjugate is an anti-HER2 antibody.
[13] The pharmaceutical composition according to [12], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2 or an antibody comprising a heavy chain consisting of an amino acid sequence represented by SEQ ID NO: 1 and a light chain consisting of an amino acid sequence represented by SEQ ID NO: 2.
[14] The pharmaceutical composition according to [12] or [13] wherein the average number of units of the drug-linker conjugated per antibody molecule in the antibody-drug conjugate is in the range of from 7 to 8.
[15] The pharmaceutical composition according to [12] to [14] comprising,
   (i) per 20 mg of the antibody-drug conjugate,
   (ii) 25 mmol of the histidine buffer,
   (iii) 90 mg of sucrose, and
   (iv) 0.3 mg of polysorbate 80,
   wherein the pH of the composition when the antibody-drug conjugate is dissolved in water at a concentration of 20 mg/mL is 5.5.
[16] The pharmaceutical composition according to [12] to [14] comprising,
   (i) per 20 mg of the antibody-drug conjugate,
   (ii) 0.89 mg of L-histidine and 4.04 mg of L-histidine hydrochloride hydrate,
   (iii) 90 mg of sucrose, and
   (iv) 0.3 mg of polysorbate 80.
[17] The pharmaceutical composition according to any one of [12] to [14] comprising
   (i) 100 mg of the antibody-drug conjugate,
   (ii) 4.45 mg of L-histidine and 20.2 mg of L-histidine hydrochloride hydrate,
   (iii) 450 mg of sucrose, and
   (iv) 1.5 mg of polysorbate 80.
[18] The pharmaceutical composition according to [11], wherein the antibody in the antibody-drug conjugate is an anti-HER3 antibody.
[19] The pharmaceutical composition according to [18], wherein the anti-HER3 antibody is an antibody comprising a heavy chain consisting of an amino acid sequence represented by SEQ ID NO: 3 and a light chain consisting of an amino acid sequence represented by SEQ ID NO: 4, or a variant of the antibody in which a lysine residue at the carboxyl terminus of the heavy chain is deleted.
[20] The pharmaceutical composition according to [18] or [19], wherein the average number of units of the drug-linker conjugated per antibody molecule in the antibody-drug conjugate is in the range of from 7 to 8.
[21] The pharmaceutical composition according to any one of [18] to [20] comprising,
   (i) per 20 mg of the antibody-drug conjugate,
   (ii) 25 mmol of the histidine buffer,
   (iii) 90 mg of sucrose, and
   (iv) 0.3 mg of polysorbate 20,
   wherein the pH of the composition when the antibody-drug conjugate is dissolved in water at a concentration of 20 mg/mL is 5.4.
[22] The pharmaceutical composition according to any one of [18] to [20] comprising,
   (i) per 20 mg of the antibody-drug conjugate,
   (ii) 0.81 mg of L-histidine and 4.14 mg of L-histidine hydrochloride hydrate,
   (iii) 90 mg of sucrose, and
   (iv) 0.3 mg of polysorbate 20.
[23] The pharmaceutical composition according to any one of [18] to [20] comprising
   (i) 100 mg of the antibody-drug conjugate,
   (ii) 4.06 mg of L-histidine and 20.7 mg of L-histidine hydrochloride hydrate,
   (iii) 450 mg of sucrose, and
   (iv) 1.5 mg of polysorbate 20.
[24] The pharmaceutical composition according to [11], wherein the antibody in the antibody-drug conjugate is an anti-TROP2 antibody.
[25] The pharmaceutical composition according to [24], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6, or a variant of the antibody in which a lysine residue at the carboxyl terminus of the heavy chain is deleted.
[26] The pharmaceutical composition according to [24] or [25], wherein the average number of units of the drug-linker conjugated per antibody molecule in the antibody-drug conjugate is in the range of from 3 to 5.
[27] The pharmaceutical composition according to any one of [24] to [26] comprising,
   (i) per 20 mg of the antibody-drug conjugate,
   (ii) 10 mmol of the histidine buffer,
   (iii) 90 mg of sucrose, and
   (iv) 0.2 or 0.3 mg of polysorbate 80,
   wherein the pH of the composition when the antibody-drug conjugate is dissolved in water at a concentration of 20 mg/mL is 6.0.
[28] The pharmaceutical composition according to any one of [24] to [26] comprising,
   (i) per 20 mg of the antibody-drug conjugate,
   (ii) 0.78 mg of L-histidine and 1.05 mg of L-histidine hydrochloride hydrate,
   (iii) 90 mg of sucrose, and
   (iv) 0.2 or 0.3 mg of polysorbate 80.
[29] The pharmaceutical composition according to any one of [24] to [26] comprising
   (i) 100 mg of the antibody-drug conjugate,
   (ii) 3.88 mg of L-histidine and 5.26 mg of L-histidine hydrochloride hydrate,
   (iii) 450 mg of sucrose, and
   (iv) 1.0 or 1.5 mg of polysorbate 80.
[30] The pharmaceutical composition according to [11], wherein the antibody in the antibody-drug conjugate is an anti-B7-H3 antibody.
[31] The pharmaceutical composition according to [30], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which a lysine residue at the carboxyl terminus of the heavy chain is deleted.
[32] The pharmaceutical composition according to [30] or [31], wherein the average number of units of the drug-linker conjugated per antibody molecule in the antibody-drug conjugate is in the range of from 3 to 5.
[33] The pharmaceutical composition according to any one of [30] to [32] comprising,
   (i) per 20 mg of the antibody-drug conjugate,
   (ii) 10 mmol of a histidine buffer,
   (iii) 90 mg of sucrose, and
   (iv) 0.2 or 0.3 mg of polysorbate 20,
   wherein the pH of the composition when the antibody-drug conjugate is dissolved in water at a concentration of 20 mg/mL is 5.9.
[34] The pharmaceutical composition according to any one of [30] to [32] comprising,
   (i) per 20 mg of the antibody-drug conjugate,
   (ii) 0.65 mg of L-histidine and 1.22 mg of L-histidine hydrochloride hydrate,
   (iii) 90 mg of sucrose, and
   (iv) 0.2 or 0.3 mg of polysorbate 20.
[35] The pharmaceutical composition according to any one of [30] to [32] comprising
   (i) 100 mg of the antibody-drug conjugate,
   (ii) 3.23 mg of L-histidine and 6.12 mg of L-histidine hydrochloride hydrate,
   (iii) 450 mg of sucrose, and
   (iv) 1.0 or 1.5 mg of polysorbate 20.
[36] The pharmaceutical composition according to [11], wherein the antibody in the antibody-drug conjugate is an anti-GPR20 antibody.
[37] The pharmaceutical composition according to [36], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10, or a variant of the antibody in which a lysine residue at the carboxyl terminus of the heavy chain is deleted.
[38] The pharmaceutical composition according to [36] or [37], wherein the average number of units of the drug-linker conjugated per antibody molecule in the antibody-drug conjugate is in the range of from 7 to 8.
[39] The pharmaceutical composition according to any one of [36] to [38] comprising,
   (i) per 20 mg of the antibody-drug conjugate,
   (ii) 10 mmol of the histidine buffer,
   (iii) 90 mg of sucrose, and
   (iv) 0.3 mg of polysorbate 80,
   wherein the pH of the composition when the antibody-drug conjugate is dissolved in water at a concentration of 20 mg/mL is 5.4.
[40] The pharmaceutical composition according to any one of [36] to [38] comprising,
   (i) per 20 mg of the antibody-drug conjugate,
   (ii) 0.32 mg of L-histidine and 1.66 mg of L-histidine hydrochloride hydrate,
   (iii) 90 mg of sucrose, and
   (iv) 0.3 mg of polysorbate 80.
[41] The pharmaceutical composition according to any one of [36] to [38] comprising
   (i) 100 mg of the antibody-drug conjugate,
   (ii) 1.62 mg of L-histidine and 8.29 mg of L-histidine hydrochloride hydrate,
   (iii) 450 mg of sucrose, and
   (iv) 1.5 mg of polysorbate 80.
[42] A pharmaceutical composition comprising,
   (i) per 20 mg of an antibody-drug conjugate,
   (ii) 0.89 mg of L-histidine and 4.04 mg of L-histidine hydrochloride hydrate,
   (iii) 90 mg of sucrose, and
   (iv) 0.3 mg of polysorbate 80,
   wherein the antibody-drug conjugate is an antibody-drug conjugate in which a drug-linker represented by the following formula: 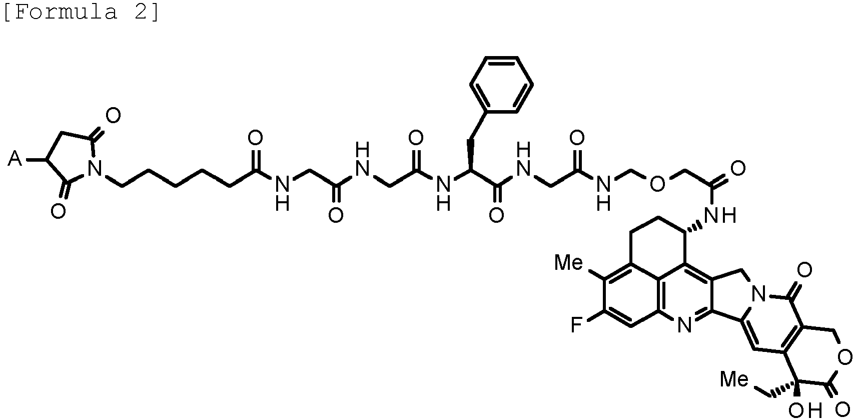 wherein A represents a connecting position to an antibody, is conjugated to the antibody via a thioether bond.
[43] The pharmaceutical composition according to [42] comprising
   (i) 100 mg of the antibody-drug conjugate,
   (ii) 4.45 mg of L-histidine and 20.2 mg of L-histidine hydrochloride hydrate,
   (iii) 450 mg of sucrose, and
   (iv) 1.5 mg of polysorbate 80.
[44] The pharmaceutical composition according to [42] or [43], wherein the average number of units of the drug-linker conjugated per antibody molecule in the antibody-drug conjugate is in the range of from 7 to 8.
[45] The pharmaceutical composition according to any one of [42] to [44], wherein the antibody in the antibody-drug conjugate is an anti-HER2 antibody.
[46] The pharmaceutical composition according to [45], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[47] The pharmaceutical composition according to [45], wherein the anti-HER2 antibody is an antibody comprising a heavy chain consisting of the amino acid sequence represented by SEQ ID NO: 1 and a light chain consisting of the amino acid sequence represented by SEQ ID NO: 2.
[48] A pharmaceutical composition comprising,
   (i) per 20 mg of an antibody-drug conjugate,
   (ii) 0.81 mg of L-histidine and 4.14 mg of L-histidine hydrochloride hydrate,
   (iii) 90 mg of sucrose, and
   (iv) 0.3 mg of polysorbate 20,
   wherein the antibody-drug conjugate is an antibody-drug conjugate in which a drug-linker represented by the following formula: 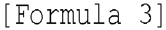 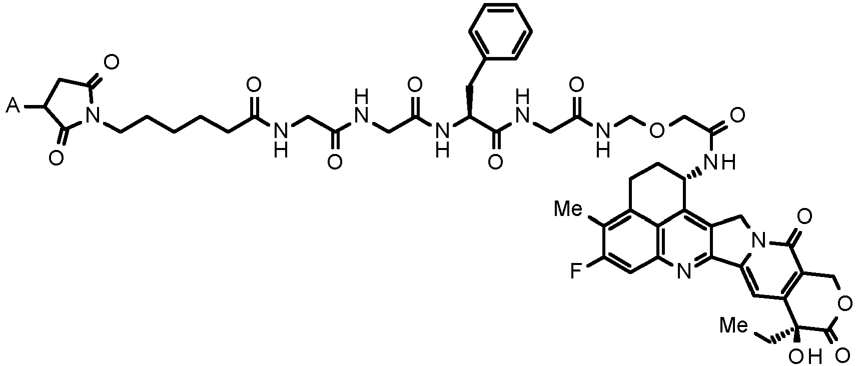 wherein A represents a connecting position to an antibody, is conjugated to the antibody via a thioether bond.
[49] The pharmaceutical composition according to [48] comprising
   (i) 100 mg of the antibody-drug conjugate,
   (ii) 4.06 mg of L-histidine and 20.7 mg of L-histidine hydrochloride hydrate,
   (iii) 450 mg of sucrose, and
   (iv) 1.5 mg of polysorbate 20.
[50] The pharmaceutical composition according to [48] or [49], wherein the average number of units of the drug-linker conjugated per antibody molecule in the antibody-drug conjugate is in the range of from 7 to 8.
[51] The pharmaceutical composition according to any one of [48] to [50], wherein the antibody in the antibody-drug conjugate is an anti-HER3 antibody.
[52] The pharmaceutical composition according to [51], wherein the anti-HER3 antibody is an antibody comprising a heavy chain consisting of the amino acid sequence represented by SEQ ID NO: 3 and a light chain consisting of the amino acid sequence represented by SEQ ID NO: 4.
[53] The pharmaceutical composition according to [52], wherein the anti-HER3 antibody lacks a lysine residue at the carboxyl terminus of the heavy chain.
[54] A pharmaceutical composition comprising,
   (i) per 20 mg of the antibody-drug conjugate,
   (ii) 0.78 mg of L-histidine and 1.05 mg of L-histidine hydrochloride hydrate,
   (iii) 90 mg of sucrose, and
   (iv) 0.2 or 0.3 mg of polysorbate 80,
   wherein the antibody-drug conjugate is an antibody-drug conjugate in which a drug-linker represented by the following formula: 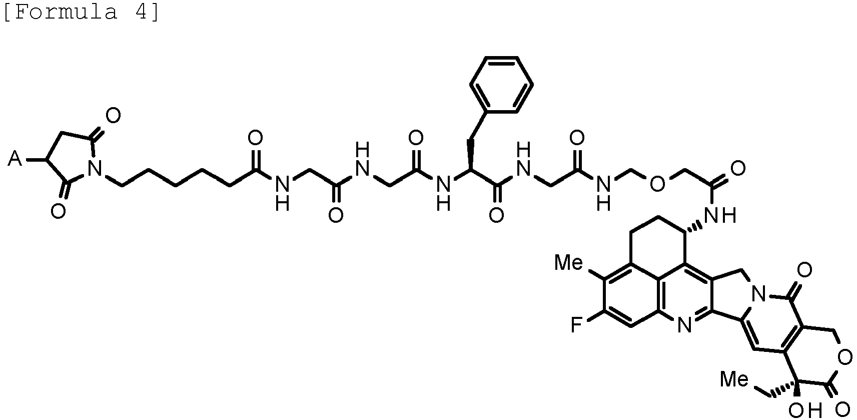 wherein A represents a connecting position to an antibody, is conjugated to the antibody via a thioether bond.
[55] The pharmaceutical composition according to [54] comprising
   (i) 100 mg of the antibody-drug conjugate,
   (ii) 3.88 mg of L-histidine and 5.26 mg of L-histidine hydrochloride hydrate,
   (iii) 450 mg of sucrose, and
   (iv) 1.0 or 1.5 mg of polysorbate 80.
[56] The pharmaceutical composition according to [54] or [55], wherein the average number of units of the drug-linker conjugated per antibody molecule in the antibody-drug conjugate is in the range of from 3 to 5.
[57] The pharmaceutical composition according to any one of [54] to [56], wherein the antibody in the antibody-drug conjugate is an anti-TROP2 antibody.
[58] The pharmaceutical composition according to [57], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[59] The pharmaceutical composition according to [58], wherein the anti-TROP2 antibody lacks a lysine residue at the carboxyl terminus of the heavy chain.
[60] A pharmaceutical composition comprising,
   (i) per 20 mg of the antibody-drug conjugate,
   (ii) 0.65 mg of L-histidine and 1.22 mg of L-histidine hydrochloride hydrate,
   (iii) 90 mg of sucrose, and
   (iv) 0.2 or 0.3 mg of polysorbate 20,
   wherein, the antibody-drug conjugate is an antibody-drug conjugate in which a drug-linker represented by the following formula: 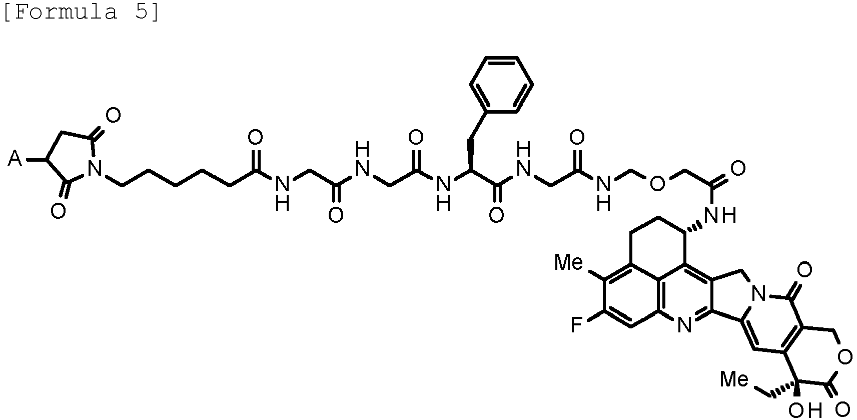 wherein A represents a connecting position to an antibody, is conjugated to the antibody via a thioether bond.
[61] The pharmaceutical composition according to [60] comprising
   (i) 100 mg of the antibody-drug conjugate,
   (ii) 3.23 mg of L-histidine and 6.12 mg of L-histidine hydrochloride hydrate,
   (iii) 450 mg of sucrose, and
   (iv) 1.0 or 1.5 mg of polysorbate 20.
[62] The pharmaceutical composition according to [60] or [61], wherein the average number of units of the drug-linker conjugated per antibody molecule in the antibody-drug conjugate is in the range of from 3 to 5.
[63] The pharmaceutical composition according to any one of [60] to [62], wherein the antibody in the antibody-drug conjugate is an anti-B7-H3 antibody.
[64] The pharmaceutical composition according to [63], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[65] The pharmaceutical composition according to [64], wherein the anti-B7-H3 antibody lacks a lysine residue at the carboxyl terminus of heavy chain.
[66] A pharmaceutical composition comprising,
   (i) per 20 mg of the antibody-drug conjugate,
   (ii) 0.32 mg of L-histidine and 1.66 mg of L-histidine hydrochloride hydrate,
   (iii) 90 mg of sucrose, and
   (iv) 0.3 mg of polysorbate 80
   wherein, the antibody-drug conjugate is an antibody-drug conjugate in which a drug-linker represented by the following formula: 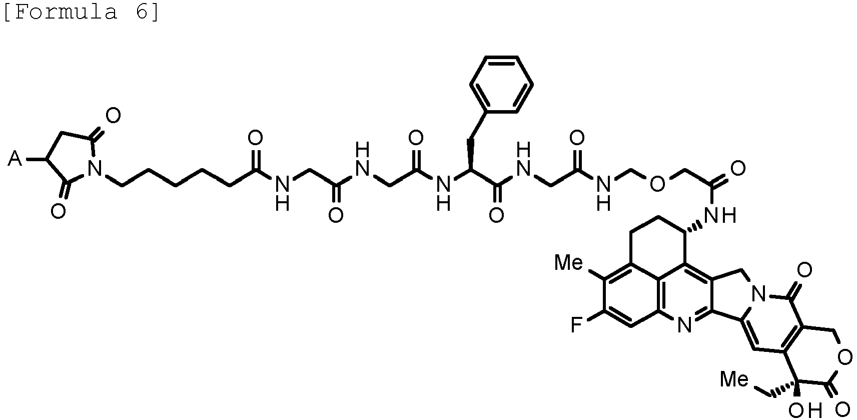 wherein A represents a connecting position to an antibody, is conjugated to the antibody via a thioether bond.
[67] The pharmaceutical composition according to [66] comprising
   (i) 100 mg of the antibody-drug conjugate,
   (ii) 1.62 mg of L-histidine and 8.29 mg of L-histidine hydrochloride hydrate,
   (iii) 450 mg of sucrose, and
   (iv) 1.5 mg of polysorbate 80.
[68] The pharmaceutical composition according to [66] or [67], wherein the average number of units of the drug-linker conjugated per antibody molecule in the antibody-drug conjugate is in the range of from 7 to 8.
[69] The pharmaceutical composition according to any one of [66] to [68], wherein the antibody in the antibody-drug conjugate is an anti-GPR20 antibody.
[70] The pharmaceutical composition according to [69], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.
[71] The pharmaceutical composition according to [70], wherein the anti-GPR20 antibody lacks a lysine residue at the carboxyl terminus of the heavy chain.
[72] The pharmaceutical composition according to any one of [1] to [71], wherein the composition is in the form of an injection.
[73] The pharmaceutical composition according to [72], wherein the composition is in the form of an aqueous injection.
[74] The pharmaceutical composition according to [73], wherein the concentration of the antibody-drug conjugate is 20 mg/mL.
[75] The pharmaceutical composition according to [73] or [74], wherein the composition is frozen.
[76] The pharmaceutical composition according to [72], wherein the composition is in the form of a lyophilized injection.
[77] The pharmaceutical composition according to [76], wherein the composition is stored in a brown vial.
[78] The pharmaceutical composition according to any one of [1] to [77], wherein the composition is for treating cancer.
[79] A method for producing the pharmaceutical composition according to [76], comprising the steps of
   (1) preparing an aqueous solution comprising predetermined amounts of
      (i) an antibody-drug conjugate,
      (ii) a histidine buffer,
      (iii) sucrose or trehalose, and
      (iv) a surfactant,
   (2) if necessary, adjusting the pH of the aqueous solution to a predetermined value, and then,
   (3) lyophilizing the aqueous solution.
[80] The production method according to [79], wherein the step of lyophilizing the aqueous solution comprises a process of annealing at a shelf temperature which is near the eutectic point of the aqueous solution,
   wherein near the eutectic point indicates the range of from a temperature which is 1.5°C lower than the eutectic point to a temperature which is 1.5°C higher than the eutectic point.
[81] The production method according to [80], wherein the step of lyophilizing the aqueous solution comprises a process of annealing at a shelf temperature which is same as the eutectic point of the aqueous solution.
[82] The production method according to [80] or [81], characterized in that the time for a process of primary drying is shortened compared with that when annealing at a shelf temperature which is 5°C lower than the eutectic point is performed.
[83] The production method according to any one of [80] to [82], characterized in that a lyophilized cake having less shrinkage is obtained compared with that when annealing at a shelf temperature which is 5°C lower than the eutectic point is performed.
[84] The production method according to [79], wherein the step of lyophilizing the aqueous solution comprises a process of performing annealing at a shelf temperature of -4 to -1°C.
[85] The production method according to [79], wherein the step of lyophilizing the aqueous solution comprises a process of performing annealing at a shelf temperature of -4 to -2°C.
[86] The production method according to [79], wherein the step of lyophilizing the aqueous solution comprises a process of performing annealing at a shelf temperature of -2.5°C.
[87] The production method according to any one of [84] to [86], wherein the step of lyophilizing the aqueous solution further comprises a process of primary drying at a shelf temperature of -5 to 5°C under a vacuum of 5 to 15 Pa.
[88] The production method according to any one of [84] to [86], wherein the step of lyophilizing the aqueous solution further comprises a process of primary drying at a shelf temperature of 0°C under a vacuum of 10 Pa.
[89] The production method according to [87] or [88], wherein the step of lyophilizing the aqueous solution further comprises a process of secondary drying at a shelf temperature of 40 to 50°C under a vacuum of 5 to 15 Pa.
[90] The production method according to [87] or [88], wherein the step of lyophilizing the aqueous solution further comprises a process of secondary drying at a shelf temperature of 45°C under a vacuum of 10 Pa.
[91] A kit comprising the pharmaceutical composition according to [42] or [43], and water for injection.
[92] The kit according to [91], wherein the water for injection is stored in an ampoule.
[93] A method for producing a lyophilized injection comprising a process of annealing an aqueous solution containing sucrose or trehalose at a shelf temperature which is near the eutectic point of the aqueous solution, wherein near the eutectic point indicates the range of from a temperature which is 1.5°C lower than the eutectic point to a temperature which is 1.5°C higher than the eutectic point.
[94] A method for producing a lyophilized injection comprising a process of annealing an aqueous solution containing sucrose or trehalose at a shelf temperature which is same as the eutectic point of the aqueous solution.

### Advantageous Effects of Invention

The present invention can provide a pharmaceutical composition (especially in the forms of an aqueous injection and a lyophilized injection) which suppresses the formation of aggregates and the generation of decomposition products, and also can provide an efficient lyophilizing method from an aqueous solution to a lyophilized injection, wherein a specific antibody-drug conjugate is included.

### Brief Description of Drawings

[Figure 1] Figure 1 is a diagram showing an amino acid sequence of a heavy chain of an anti-HER2 antibody (SEQ ID NO: 1).
[Figure 2] Figure 2 is a diagram showing an amino acid sequence of a light chain of an anti-HER2 antibody (SEQ ID NO: 2).
[Figure 3] Figure 3 is a diagram related to excipient screening. The abscissa depicts time course in each formulation and the ordinate depicts content of the aggregates.
[Figure 4] Figure 4 is a diagram related to buffer screening. The abscissa depicts time course in each formulation and the ordinate depicts content of the aggregates.
[Figure 5] Figure 5 is a diagram related to pH screening. The abscissa depicts time course in each formulation and the ordinate depicts content of the aggregates.
[Figure 6] Figure 6 is a diagram related to pH screening. The abscissa depicts time course in each formulation and the ordinate depicts content of NPI.
[Figure 7] Figure 7 is a diagram related to surfactant screening. The abscissa depicts time course in each formulation and the ordinate depicts content ratio of the number of insoluble microparticles less than 10 mm. In the Figure, "PS" means polysorbate, "PS20" means polysorbate 20, and "PS80" means polysorbate 80.
[Figure 8] Figure 8 is a diagram related to a study on vials. The abscissa depicts amount of light irradiation and the ordinate depicts content of the aggregates.
[Figure 9] Figure 9 is a diagram related to a study on vials. The abscissa depicts amount of light irradiation and the ordinate depicts content of IoP.
[Figure 10] Figure 10 is a contour plot of the product temperature of aqueous solution (1) when varying the shelf temperature and the chamber vacuum degree in a primary drying process.
[Figure 11] Figure 11 is a contour plot of the drying time of aqueous solution (1) when varying the shelf temperature and the chamber vacuum degree in the primary drying process.
[Figure 12] Figure 12 is a chart showing the product temperatures (during annealing) and shapes of lyophilized cakes when performing annealing with shelf temperatures of -7 to 0.5°C and lyophilizing.
[Figure 13] Figure 13 is a diagram showing thermal denaturation of antibody-drug conjugate (1) by a capillary differential scanning calorimeter.

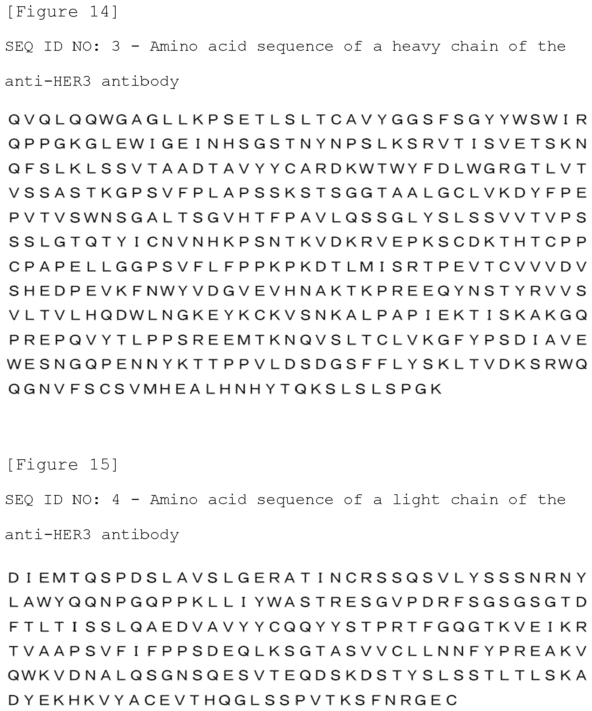

[Figure 14] Figure 14 is a diagram showing an amino acid sequence of a heavy chain of an anti-HER3 antibody (SEQ ID NO: 3).
[Figure 15] Figure 15 is a diagram showing an amino acid sequence of a light chain of an anti-HER3 antibody (SEQ ID NO: 4).

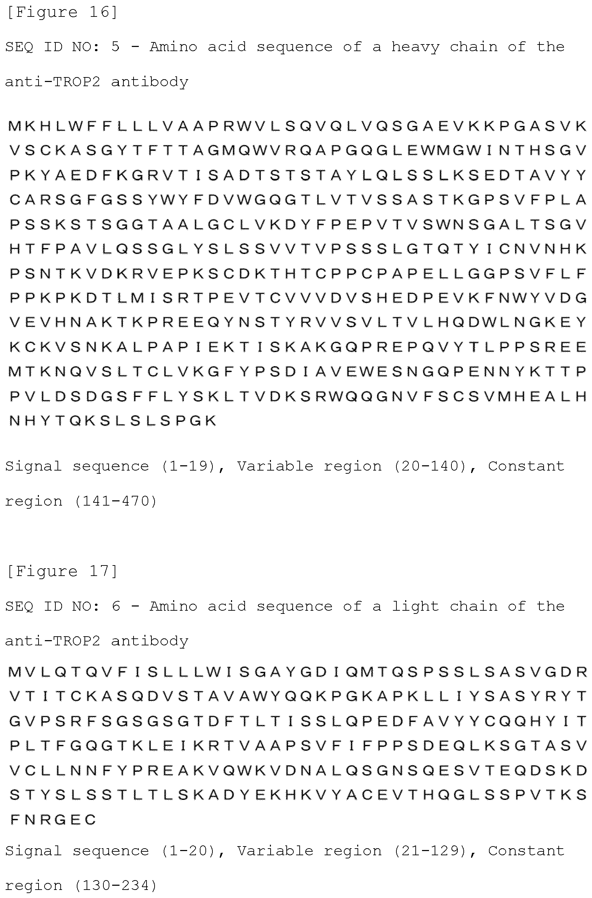

[Figure 16] Figure 16 is a diagram showing an amino acid sequence of a heavy chain of an anti-TROP2 antibody (SEQ ID NO: 5).
[Figure 17] Figure 17 is a diagram showing an amino acid sequence of a light chain of an anti-TROP2 antibody (SEQ ID NO: 6).

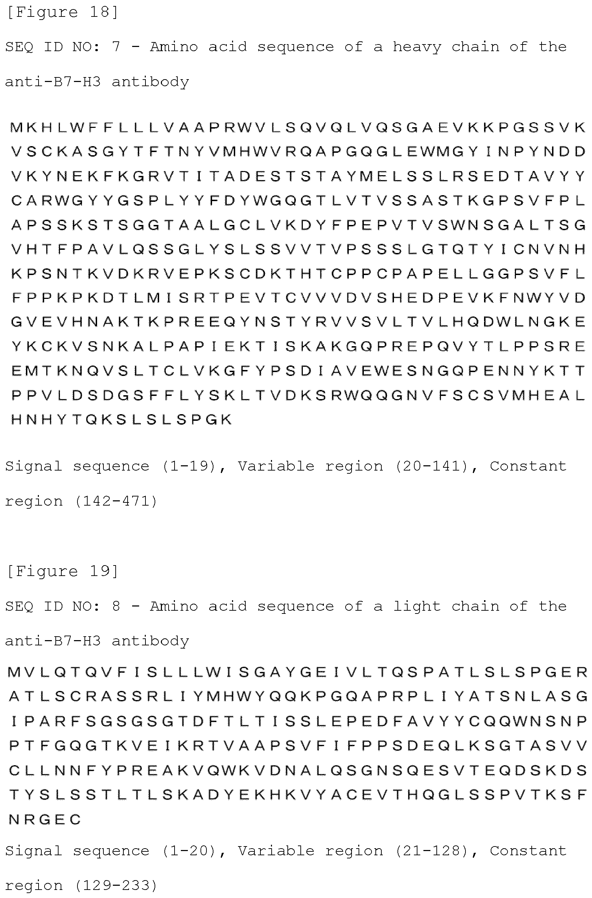

[Figure 18] Figure 18 is a diagram showing an amino acid sequence of a heavy chain of an anti-B7-H3 antibody (SEQ ID NO: 7).
[Figure 19] Figure 19 is a diagram showing an amino acid sequence of a light chain of an anti-B7-H3 antibody (SEQ ID NO: 8).

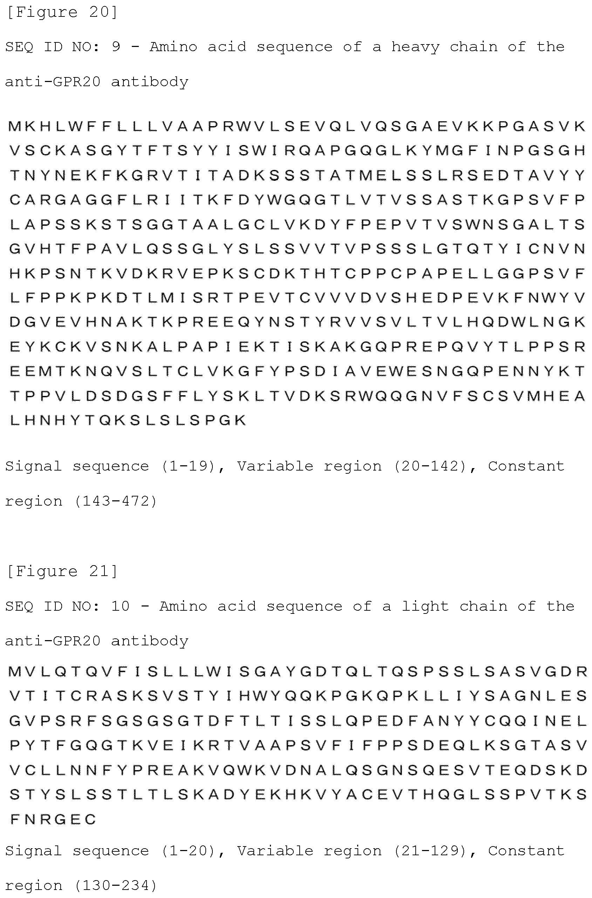

[Figure 20] Figure 20 is a diagram showing an amino acid sequence of a heavy chain of an anti-GPR20 antibody (SEQ ID NO: 9).
[Figure 21] Figure 21 is a diagram showing an amino acid sequence of a light chain of an anti-GPR20 antibody (SEQ ID NO: 10).

### Description of Embodiments

Hereinafter, preferred modes for carrying out the present invention are described with reference to the drawings. The embodiments described below are given merely for illustrating one example of a typical embodiment of the present invention and are not intended to limit the scope of the present invention.

### [Antibody-drug conjugate]

The antibody-drug conjugate used in the present invention is an antibody-drug conjugate in which a drug-linker represented by the following formula: 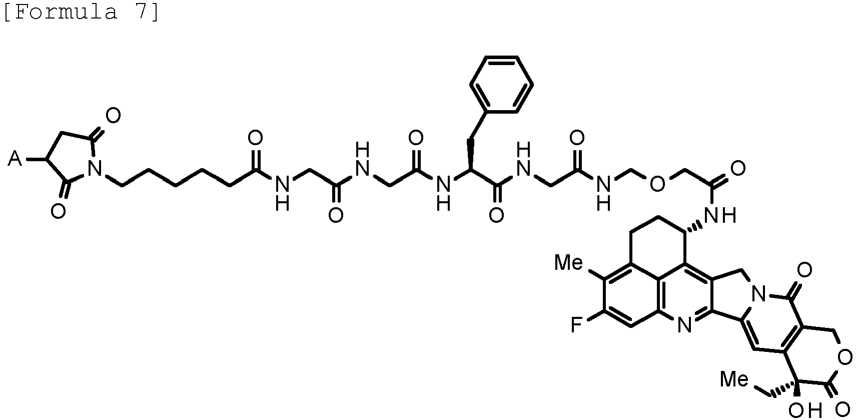 wherein A represents a connecting position to an antibody, is conjugated to the antibody via a thioether bond.

In the present invention, the partial structure consisting of a linker and a drug in the antibody-drug conjugate is referred to as a "drug-linker". The drug-linker is connected to a thiol group (in other words, the sulfur atom of a cysteine residue) formed at an interchain disulfide bond site (two sites between heavy chains, and two sites between a heavy chain and a light chain) of the antibody.

The drug-linker of the present invention includes exatecan (IUPAC name: (1S,9S)-1-amino-9-ethyl-5-fluoro-1,2,3,9,12,15-hexahydro-9-hydroxy-4-methyl-10H,13H-benzo[de]pyrano[3',4':6,7]indolizino[1,2-b]quinolin 13-dione, (also expressed as chemical name: (1S, 9S)-1-amino-9-ethyl-5-fluoro-2,3-dihydro-9-hydroxy-4-methyl-1H,12H-benzo[de]pyrano[3',4':6,7]indolizino[1,2-b]quinolin-10,13(9H,15H)-dione)), which is a topoisomerase I inhibitor, as a component. Exatecan is a camptothecin derivative having an antitumor effect, represented by the following formula:

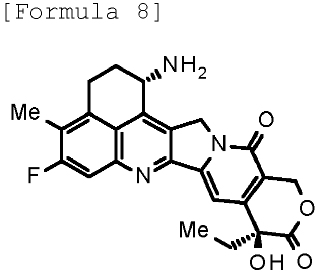

The antibody-drug conjugate used in the present invention can also be represented by the following formula: 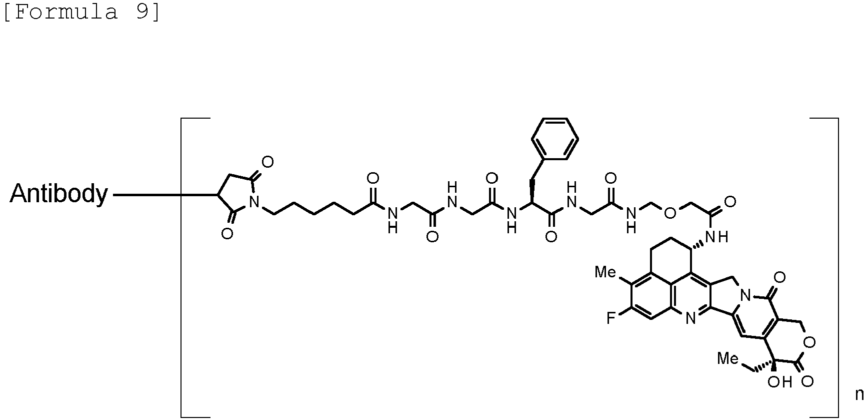 wherein, the drug-linker is conjugated to an antibody via a thioether bond. The meaning of n is the same as that of what is called the average number of conjugated drug molecules (DAR; Drug-to-Antibody Ratio), and indicates the average number of units of the drug-linker conjugated per antibody molecule.

After migrating into cancer cells, the antibody-drug conjugate used in the present invention releases the compound, represented by the following formula: 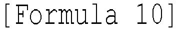 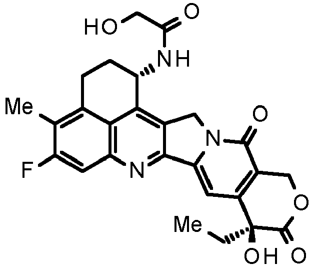 and having a topoisomerase I inhibitory effect (hereinafter, referred to as "compound (1)").

The compound (1) is inferred to be formed by decomposition of an aminal structure of the compound represented by the following formula: 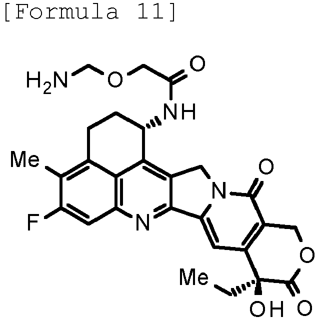 which is inferred to be formed by cleavage of the linker part of the antibody-drug conjugate used in the present invention.

The antibody-drug conjugate used in the present invention is known to have a bystander effect (Ogitani Y. et al., Cancer Science (2016) 107, 1039-1046).

The bystander effect is exerted through a process in which the antibody-drug conjugate used in the present invention is internalized in cancer cells expressing a target and the compound (1) released then exerts an antitumor effect also on cancer cells which are present therearound and not expressing the target.

### [Antibody in Antibody-drug conjugate]

The antibody in the antibody-drug conjugate used in the present invention may be derived from any species, and is preferably an antibody derived from a human, a rat, a mouse, or a rabbit. In cases where the antibody is derived from species other than human species, it is preferably chimerized or humanized using a well-known technique. The antibody of the present invention may be a polyclonal antibody or a monoclonal antibody and is preferably a monoclonal antibody.

The antibody in the antibody-drug conjugate in the present invention is an antibody preferably having a property of being capable of targeting cancer cells, and is preferably an antibody possessing, for example, the property of recognizing a cancer cell, the property of binding to a cancer cell, the property of internalizing in a cancer cell, and/or cytocidal activity against a cancer cell.

The binding activity of the antibody against cancer cells can be confirmed using flow cytometry. The internalization of the antibody into cancer cells can be confirmed using (1) an assay of visualizing an antibody incorporated in cells by a fluorescence microscope using a secondary antibody (fluorescently labeled) binding to the therapeutic antibody (Cell Death and Differentiation (2008) 15, 751-761), (2) an assay of measuring a fluorescence intensity incorporated in cells using a secondary antibody (fluorescently labeled) binding to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). As the immunotoxin, a recombinant complex protein of a diphtheria toxin catalytic region and protein G may be used.

The antitumor activity of the antibody can be confirmed in vitro by determining inhibitory activity against cell growth. For example, a cancer cell line overexpressing a target protein for the antibody is cultured, and the antibody is added into the culture system at varying concentrations to determine inhibitory activity against focus formation, colony formation, and spheroid growth. The antitumor activity can be confirmed in vivo, for example, by administering the antibody to a nude mouse with a transplanted cancer cell line highly expressing the target protein, and determining change in the cancer cell.

Since the compound conjugated in the antibody-drug conjugate exerts an antitumor effect, it is preferred but not essential that the antibody itself should have an antitumor effect. For the purpose of specifically and selectively exerting the cytotoxic activity of the antitumor compound against cancer cells, it is important and also preferred that the antibody should have the property of internalizing to migrate into cancer cells.

The antibody in the antibody-drug conjugate used in the present invention can be obtained by a procedure known in the art. For example, the antibody of the present invention can be obtained using a method usually carried out in the art, which involves immunizing animals with an antigenic polypeptide and collecting and purifying antibodies produced in vivo. The origin of the antigen is not limited to humans, and the animals may be immunized with an antigen derived from a non-human animal such as a mouse, a rat and the like. In this case, the cross-reactivity of antibodies binding to the obtained heterologous antigen with human antigens can be tested to screen for an antibody applicable to a human disease.

Alternatively, antibody-producing cells which produce antibodies against the antigen are fused with myeloma cells according to a method known in the art (e.g., Kohler and Milstein, Nature (1975) 256, p. 495-497; and Kennet, R. ed., Monoclonal Antibodies, p. 365-367, Plenum Press, N.Y. (1980)) to establish hybridomas, from which monoclonal antibodies can in turn be obtained.

The antigen can be obtained by genetically engineering host cells to produce a gene encoding the antigenic protein. Specifically, vectors that permit expression of the antigen gene are prepared and transferred to host cells so that the gene is expressed. The antigen thus expressed can be purified. The antibody can also be obtained by a method for immunizing animals with the above-described genetically engineered antigen-expressing cells or a cell line expressing the antigen.

The antibody in the antibody-drug conjugate used in the present invention is preferably a recombinant antibody obtained by artificial modification for the purpose of decreasing heterologous antigenicity to humans such as a chimeric antibody or a humanized antibody, or is preferably an antibody having only the gene sequence of an antibody derived from a human, that is, a human antibody. These antibodies can be produced using a known method.

As the chimeric antibody, an antibody in which antibody variable and constant regions are derived from different species, for example, a chimeric antibody in which a mouse- or rat-derived antibody variable region is connected to a human-derived antibody constant region can be exemplified (Proc. Natl. Acad. Sci. U.S.A., 81, 6851-6855, (1984)).

As the humanized antibody, an antibody obtained by integrating only the complementarity determining region (CDR) of a heterologous antibody into a human-derived antibody (Nature (1986) 321, pp. 522-525), and an antibody obtained by grafting a part of the amino acid residues of the framework of a heterologous antibody as well as the CDR sequence of the heterologous antibody to a human antibody by a CDR-grafting method (WO 90/07861), and an antibody humanized using a gene conversion mutagenesis strategy (U.S. Patent No. 5821337) can be exemplified.

As the human antibody, an antibody generated by using a human antibody-producing mouse having a human chromosome fragment including genes of a heavy chain and light chain of a human antibody (see, for example, Tomizuka, K. et al., Nature Genetics (1997) 16, p.133-143; Kuroiwa, Y. et. al., Nucl. Acids Res. (1998) 26, p.3447-3448; Yoshida, H. et. al., Animal Cell Technology: Basic and Applied Aspects vol.10, p.69-73 (Kitagawa, Y., Matsuda, T. and Iijima, S. eds.), Kluwer Academic Publishers, 1999; Tomizuka, K. et. al., Proc. Natl. Acad. Sci. USA (2000) 97, p.722-727) can be exemplified. Alternatively, the antibody obtained by screening from a human antibody library by phage display (see, for example, Wormstone, I. M. et al, Investigative Ophthalmology & Visual Science (2002) 43(7), p. 2301-2308; Carmen, S. et al., Briefings in Functional Genomics and Proteomics (2002), 1(2), p.189-203;. Siriwardena, D. et al, Ophthalmology (2002) 109(3), p.427-431) can be exemplified.

In the present invention, modified variants of the antibody in the antibody-drug conjugate used in the present invention are also included. The modified variant refers to a variant obtained by subjecting the antibody according to the present invention to chemical or biological modification. Examples of the chemically modified variant include variants including a linkage of a chemical moiety to an amino acid skeleton, variants including a linkage of a chemical moiety to an N-linked or O-linked carbohydrate chain, etc. Examples of the biologically modified variant include variants obtained by post-translational modification (such as N-linked or O-linked glycosylation, N- or C-terminal processing, deamidation, isomerization of aspartic acid, or oxidation of methionine), and variants in which a methionine residue has been added to the N terminus by being expressed in a prokaryotic host cell. Further, an antibody labeled so as to enable the detection or isolation of the antibody or an antigen according to the present invention, for example, an enzyme-labeled antibody, a fluorescence-labeled antibody, and an affinity-labeled antibody are also included in the meaning of the modified variant. Such a modified variant of the antibody according to the present invention is useful for improving the stability and blood retention of the antibody, reducing the antigenicity thereof, detecting or isolating an antibody or an antigen, and so on.

Furthermore, by regulating the modification of a glycan which is linked to the antibody according to the present invention (glycosylation, defucosylation, etc.), it is possible to enhance antibody-dependent cellular cytotoxic activity. As the technique for regulating the modification of a glycan of antibodies, WO 99/54342, WO 00/61739, WO 02/31140, etc. are known. However, the technique is not limited thereto. In the antibody according to the present invention, antibodies in which the modification of a glycan is regulated are also included.

It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (the activation of complement, antibody-dependent cellular cytotoxicity, etc.) of the antibody. Therefore, in the antibody according to the present invention, antibodies subjected to such modification and functional fragments of the antibody are also included, and deletion variants in which one or two amino acids have been deleted at the carboxyl terminus of the heavy chain, variants obtained by amidation of deletion variants (for example, a heavy chain in which the carboxyl terminal proline residue has been amidated), and the like are also included. The type of deletion variant having a deletion at the carboxyl terminus of the heavy chain of the antibody according to the present invention is not limited to the above variants as long as the antigen-binding affinity and the effector function are conserved. The two heavy chains constituting the antibody according to the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the antibody according to the present invention and the culture conditions; however, an antibody in which one amino acid residue at the carboxyl terminus has been deleted in both of the two heavy chains in the antibody according to the present invention can be preferably exemplified.

As isotypes of the antibody according to the present invention, for example, IgG (IgG1, IgG2, IgG3, IgG4) can be exemplified, and IgG1 or IgG2 can be exemplified preferably.

Examples of antibodies in the antibody-drug conjugate used in the present invention can include, but are not particularly limited to, an anti-HER2 antibody, an anti-HER3 antibody, an anti-TROP2 antibody, an anti-B7-H3 antibody, an anti-CD3 antibody, an anti-CD30 antibody, an anti-CD33 antibody, an anti-CD37 antibody, an anti-CD56 antibody, an anti-CD98 antibody, an anti-DR5 antibody, an anti-EGFR antibody, an anti-EPHA2 antibody, an anti-FGFR2 antibody, an anti-FGFR4 antibody, an anti-FOLR1 antibody, an anti-VEGF antibody, and an anti-GPR20 antibody, and preferably an anti-HER2 antibody, an anti-HER3 antibody, an anti-TROP2 antibody, an anti-B7-H3 antibody, and an anti-GPR20 antibody can be exemplified.

In the present invention, the term "anti-HER2 antibody" refers to an antibody which specifically binds to HER2 (Human Epidermal Growth Factor Receptor Type 2; ErbB-2), and preferably has an activity of internalizing in HER2-expressing cells by binding to HER2.

Examples of the anti-HER2 antibody include trastuzumab (U.S. Patent No. 5821337) and pertuzumab (International Publication No. WO 01/00245), and trastuzumab can be preferably exemplified.

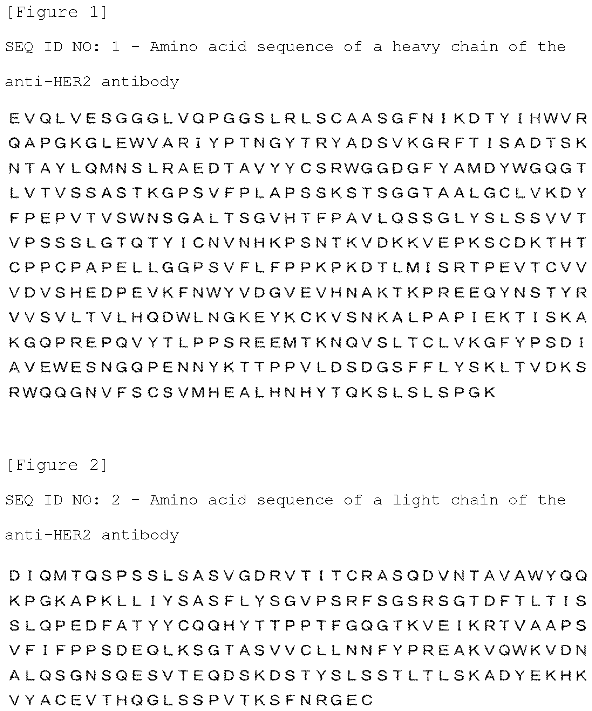

In the present invention, the term "trastuzumab" is a humanized anti-HER2 monoclonal antibody comprising a heavy chain consisting of an amino acid sequence consisting of amino acid residues 1 to 449 of SEQ ID NO: 1 (Figure 1) and a light chain consisting of an amino acid sequence consisting of amino acid residues 1 to 214 of SEQ ID NO: 2 (Figure 2).

In the present invention, the term "anti-HER3 antibody" refers to an antibody which specifically binds to HER3 (Human Epidermal Growth Factor Receptor Type 3; ErbB-3), and preferably has an activity of internalizing in HER3-expressing cells by binding to HER3 on the surface of the HER3-expressing cells.

Examples of the anti-HER3 antibody include Patritumab (U3-1287), U1-59 (International Publication No. WO 2007/077028), MM-121 (seribantumab), an anti-ERBB3 antibody described in International Publication No. WO 2008/100624, RG-7116 (Lumretuzumab) and LJM-716 (Elgemtumab), and Patritumab and U1-59 can be preferably exemplified.

In the present invention, the term "anti-TROP2 antibody" refers to an antibody which specifically binds to TROP2 (TACSTD2: Tumor-associated calcium signal transducer 2; EGP-1), and preferably has an activity of internalizing in TROP2-expressing cells by binding to TROP2.

Examples of the anti-TROP2 antibody include hTINA1-H1L1 (International Publication No. WO 2015/098099).

In the present invention, the term "anti-B7-H3 antibody" refers to an antibody which specifically binds to B7-H3 (B cell antigen #7 homolog 3; PD-L3; CD276), and preferably has an activity of internalizing in B7-H3-expressing cells by binding to B7-H3.

Examples of the anti-B7-H3 antibody include M30-H1-L4 (International Publication No. WO 2014/057687).

In the present invention, the term "anti-GPR20 antibody" refers to an antibody which specifically binds to GPR20 (G Protein-coupled receptor 20), and preferably has an activity of internalizing in GPR20-expressing cells by binding to GPR20.

Examples of the anti-GPR20 antibody include h046-H4e/L7 (International Publication No. WO 2018/135501).

### [Drug-linker intermediate for use in the production of antibody-drug conjugate]

A drug-linker intermediate for use in the production of the antibody-drug conjugate according to the present invention is represented by the following formula.

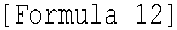

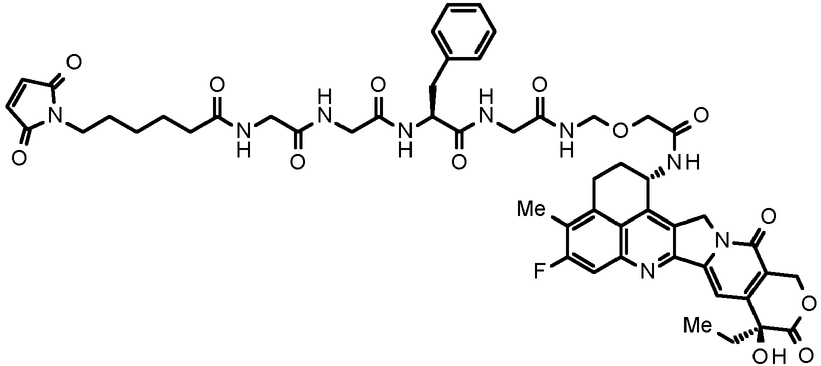

The drug-linker intermediate can be produced with reference to descriptions in International Publication No. WO 2014/057687, International Publication No. WO 2015/098099, International Publication No. WO 2015/115091, International Publication No. WO 2015/155998, and so on.

### [Conjugation between the antibody and the drug-linker intermediate]

The antibody-drug conjugate used in the present invention can be produced by having the above-described drug-linker intermediate react with an antibody having a thiol group (alternatively referred to as a sulfhydryl group) .

The antibody having a sulfhydryl group can be obtained by a method well known in the art (Hermanson, G. T, Bioconjugate Techniques, pp.56-136, pp.456-493, Academic Press(1996)). For example, by using 0.3 to 3 molar equivalents of a reducing agent such as tris(2-carboxyethyl)phosphine hydrochloride (TCEP) per interchain disulfide within the antibody and reacting with the antibody in a buffer solution containing a chelating agent such as ethylenediamine tetraacetic acid (EDTA), an antibody having a sulfhydryl group with partially or completely reduced interchain disulfides within the antibody can be obtained.

Furthermore, by using 2 to 20 molar equivalents of the drug-linker intermediate per antibody having a sulfhydryl group, an antibody-drug conjugate in which 2 to 8 drug molecules are conjugated per antibody molecule can be produced.

The average number of conjugated drug molecules per antibody molecule of the antibody-drug conjugate produced can be determined, for example, by a method of calculation based on measurement of UV absorbance for the antibody-drug conjugate and the conjugation precursor thereof at two wavelengths of 280 nm and 370 nm (UV method), or a method of calculation based on quantification through HPLC measurement for fragments obtained by treating the antibody-drug conjugate with a reducing agent (HPLC method).

Conjugation between the antibody and the drug-linker intermediate and calculation of the average number of conjugated drug molecules per antibody molecule of the antibody-drug conjugate can be performed with reference to descriptions in International Publication No. WO 2014/057687, International Publication No. WO 2015/098099, International Publication No. WO 2015/115091, International Publication No. WO 2015/155998, International Publication No. WO 2018/135501, and so on.

In the present invention, the term "anti-HER2 antibody-drug conjugate" refers to an antibody-drug conjugate in which the antibody in the antibody-drug conjugate is an anti-HER2 antibody.

The anti-HER2 antibody is preferably an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2 or an antibody comprising a heavy chain consisting of an amino acid sequence represented by SEQ ID NO: 1 and a light chain consisting of an amino acid sequence represented by SEQ ID NO: 2.

The average number of units of the drug-linker conjugated per antibody molecule in the anti-HER2 antibody-drug conjugate used in the present invention is preferably 2 to 8, more preferably 3 to 8, even more preferably 7 to 8, even more preferably 7.5 to 8, and even more preferably about 8.

The anti-HER2 antibody-drug conjugate used in the present invention can be produced with reference to descriptions in International Publication No. WO 2015/115091 and so on.

In the present invention, the term "anti-HER3 antibody-drug conjugate" refers to an antibody-drug conjugate in which the antibody in the antibody-drug conjugate is an anti-HER3 antibody.

The anti-HER3 antibody is preferably an antibody comprising a heavy chain consisting of an amino acid sequence represented by SEQ ID NO: 3 and a light chain consisting of an amino acid sequence represented by SEQ ID NO: 4, or a variant of the antibody in which a lysine residue at the carboxyl terminus of the heavy chain is deleted.

The average number of units of the drug-linker conjugated per antibody molecule in the anti-HER3 antibody-drug conjugate used in the present invention is preferably 2 to 8, more preferably 3 to 8, even more preferably 7 to 8, even more preferably 7.5 to 8, and even more preferably about 8.

The anti-HER3 antibody-drug conjugate used in the present invention can be produced with reference to descriptions in International Publication No. WO 2015/155998 and so on.

In the present invention, the term "anti-TROP2 antibody-drug conjugate" refers to an antibody-drug conjugate in which the antibody in the antibody-drug conjugate is an anti-TROP2 antibody.

The anti-TROP2 antibody is preferably an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6, or a variant of the antibody in which a lysine residue at the carboxyl terminus of the heavy chain is deleted.

The average number of units of the drug-linker conjugated per antibody molecule in the anti-TROP2 antibody-drug conjugate used in the present invention is preferably 2 to 8, more preferably 3 to 5, even more preferably 3.5 to 4.5, and even more preferably about 4.

The anti-TROP2 antibody-drug conjugate used in the present invention can be produced with reference to descriptions in International Publication No. WO 2015/098099 and so on.

In the present invention, the term "anti-B7-H3 antibody-drug conjugate" refers to an antibody-drug conjugate in which the antibody in the antibody-drug conjugate is an anti-B7-H3 antibody.

The anti-B7-H3 antibody is preferably an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which a lysine residue at the carboxyl terminus of the heavy chain is deleted.

The average number of units of the drug-linker conjugated per antibody molecule in the anti-B7-H3 antibody-drug conjugate used in the present invention is preferably 2 to 8, more preferably 3 to 5, even more preferably 3.5 to 4.5, and even more preferably about 4.

The anti-B7-H3 antibody-drug conjugate used in the present invention can be produced with reference to descriptions in International Publication No. WO 2014/057687 and so on.

In the present invention, the term "anti-GPR20 antibody-drug conjugate" refers to an antibody-drug conjugate in which the antibody in the antibody-drug conjugate is an anti-GPR20 antibody.

The anti-GPR20 antibody is preferably an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10, or a variant of the antibody in which a lysine residue at the carboxyl terminus of the heavy chain is deleted.

The average number of units of the drug-linker conjugated per antibody molecule in the anti-GPR20 antibody-drug conjugate used in the present invention is preferably 2 to 8, more preferably 3 to 8, even more preferably 7 to 8, even more preferably 7.5 to 8, and even more preferably about 8.

The anti-GPR20 antibody-drug conjugate used in the present invention can be produced with reference to descriptions in International Publication No. WO 2018/135501 and so on.

### [Pharmaceutical Compositions]

Hereinafter, the pharmaceutical composition according to the present invention is described.

The pharmaceutical composition according to the present invention comprises
(i) an antibody-drug conjugate,
(ii) a histidine buffer,
(iii) sucrose or trehalose, and
(iv) a surfactant,
wherein, the antibody-drug conjugate is an antibody-drug conjugate in which a drug-linker represented by the following formula: 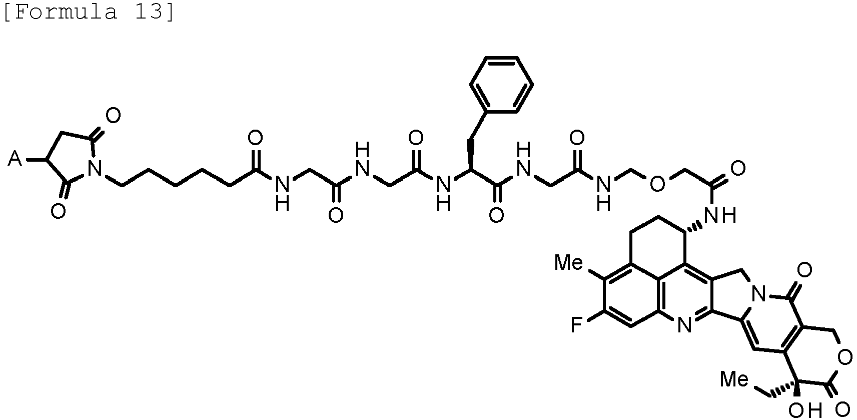 wherein A represents a connecting position to an antibody, is conjugated to the antibody via a thioether bond.

In the present invention, the term "histidine buffer" is a mixture of histidine and a salt of histidine (an acid adduct), preferably, a mixture of L-histidine and L-histidine hydrochloride (and/or a hydrate thereof). Water in which a histidine buffer is dissolved in the present invention is synonymous with a histidine buffer solution.

The amount of the histidine buffer is preferably 3 to 80 mmol per 20 mg of the antibody-drug conjugate, more preferably 10 to 80 mmol per 20 mg of the antibody-drug conjugate, even more preferably 10 to 40 mmol per 20 mg of the antibody-drug conjugate, even more preferably 10 to 25 mmol per 20 mg of the antibody-drug conjugate, and even more preferably 10 or 25 mmol per 20 mg of the antibody-drug conjugate.

It should be noted that the pH of a state where the pharmaceutical composition of the present invention is dissolved in water depends on the content ratio of histidine and salts of histidine in the histidine buffer solution. In other words, by adjusting the content ratio of histidine and salts of histidine, a pharmaceutical composition having a desired pH in a state dissolved in water can be provided.

The pH of the pharmaceutical composition of the present invention when the antibody-drug conjugate is dissolved in water at a concentration of 20 mg/mL is 4.0 to 7.0, preferably 5.0 to 6.0. Furthermore, depending on the type of antibody in the antibody-drug conjugate, a more suitable pH can be selected.

The amount of sucrose or trehalose is preferably 24 to 320 mg per 20 mg of the antibody-drug conjugate. For sucrose, the amount is more preferably 90 mg. For trehalose, when calculated as trehalose hydrate, the amount is more preferably 100 mg. Of the sucrose and trehalose, the pharmaceutical compositions of the present invention may employ sucrose more preferably.

In the present invention, the term "surfactant" refers to a substance having a hydrophilic group and a hydrophobic group and being used as one of the constituents of the pharmaceutical formulation. The surfactant in the present invention is preferably polysorbate, such as polysorbate 80 (Tween 80), polysorbate 20 (Tween20), and polysorbate 60 (Tween 60), polyoxyethylene(160) polyoxypropylene(30) glycol, polyoxyethylene hydrogenated castor oil 60, or polyoxyethylene castor oil or sodium lauryl sulfate, and more preferably polysorbate 80 or polysorbate 20.

The amount of polysorbate 80 or polysorbate 20 is, per 20 mg of the antibody-drug conjugate, preferably 0.05 to 1.6 mg, more preferably 0.1 to 1.6 mg, even more preferably 0.2 to 0.4 mg, even more preferably 0.2 to 0.3 mg, and even more preferably 0.2 or 0.3 mg.

With overall consideration of the above, the pharmaceutical compositions of the invention, is preferably,
a pharmaceutical composition comprising,
(i) per 20 mg of an antibody-drug conjugate,
(ii) 3 to 80 mmol of a histidine buffer,
(iii) 24 to 320 mg of sucrose or trehalose, and
(iv) 0.05 to 1.6 mg of polysorbate 80 or polysorbate 20,
more preferably,
a pharmaceutical composition comprising,
(i) per 20 mg of the antibody-drug conjugate,
(ii) 10 to 40 mmol of the histidine buffer,
(iii) 90 mg of sucrose or 100 mg of trehalose hydrate, and
(iv) 0.2 to 0.4 mg of polysorbate 80 or polysorbate 20,
even more preferably,
a pharmaceutical composition comprising,
(i) per 20 mg of the antibody-drug conjugate,
(ii) 10 or 25 mmol of the histidine buffer,
(iii) 90 mg of sucrose, and
(iv) 0.2 or 0.3 mg of polysorbate 80 or polysorbate 20.

When expressed as an aqueous solution comprising the antibody-drug conjugate at the concentration of 20 mg/mL, the above-described pharmaceutical composition can be rephrased as follows.

That is, the pharmaceutical composition of the present invention, is preferably,
a pharmaceutical composition comprising
(i) 20 mg/mL of the antibody-drug conjugate,
(ii) a histidine buffer,
(iii) sucrose or trehalose, and
(iv) a surfactant, and
(v) water,
more preferably,
a pharmaceutical composition comprising
(i) 20 mg/mL of the antibody-drug conjugate,
(ii) 3 to 80 mM of the histidine buffer,
(iii) 2.4 to 32% of sucrose or trehalose,
(iv) 0.005 to 0.16% of polysorbate 80 or polysorbate 20, and
(v) water,
even more preferably,
a pharmaceutical composition comprising
(i) 20 mg/mL of the antibody-drug conjugate,
(ii) 10 to 40 mM of the histidine buffer,
(iii) 9% of sucrose or 10% of trehalose,
(iv) 0.02 to 0.04% of polysorbate 80 or polysorbate 20, and
(v) water,
even more preferably,
a pharmaceutical composition comprising
(i) 20 mg/mL of the antibody-drug conjugate,
(ii) 10 or 25 mM of the histidine buffer,
(iii) 9% of sucrose, and
(iv) 0.02 or 0.03% of polysorbate 80 or polysorbate 20, and
(v) water.

In the present invention, "%" in the content of sucrose and trehalose refers to the weight% to 1 mL of water. Thus, for example, "9% of sucrose" means that it contains 90 mg of sucrose to 1 mL of water, and "10% of trehalose hydrate" means that it contains 100 mg of trehalose hydrate to 1 mL of water.

Similarly, in the present invention, "%" as to the content of polysorbate 80 and polysorbate 20 refers to the weight% to 1 mL of water. Thus, for example, "0.03% of polysorbate 80" means that it contains 0.3 mg of polysorbate 80 to 1 mL of water, and "0.03% of polysorbate 20" means that it contains 0.3 mg of polysorbate 20 to 1 mL of water.

For the pharmaceutical composition of the present invention, depending on the type of antibody in the antibody-drug conjugate, more suitable formulations can be selected.

For example, when the antibody in the antibody-drug conjugate is an anti-HER2 antibody (preferably, an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2 or an antibody comprising a heavy chain consisting of an amino acid sequence represented by SEQ ID NO: 1 and a light chain consisting of an amino acid sequence represented by SEQ ID NO: 2), the pharmaceutical composition of the present invention is preferably a pharmaceutical composition comprising,
(i) per 20 mg of the antibody-drug conjugate,
(ii) 25 mmol of a histidine buffer,
(iii) 90 mg of sucrose, and
(iv) 0.3 mg of polysorbate 80.

Furthermore, the pH of the pharmaceutical composition when the antibody-drug conjugate is dissolved in water at a concentration of 20 mg/mL is preferably 5.3 to 5.7, more preferably 5.4 to 5.6, still more preferably 5.5.

When the histidine buffer is expressed by the content of L-histidine and L-histidine hydrochloride, the above-described pharmaceutical composition when the pH thereof is 5.5 can be expressed as a pharmaceutical composition comprising,
(i) per 20 mg of the antibody-drug conjugate,
(ii) 0.89 mg of L-histidine and 4.04 mg of L-histidine hydrochloride hydrate,
(iii) 90 mg of sucrose, and
(iv) 0.3 mg of polysorbate 80.

Furthermore, when expressed as a unit formulation comprising 100 mg of the antibody-drug conjugate, the above-described pharmaceutical composition when the pH thereof is 5.5 can be expressed as a pharmaceutical composition comprising
(i) 100 mg of the antibody-drug conjugate,
(ii) 4.45 mg of L-histidine and 20.2 mg of L-histidine hydrochloride hydrate,
(iii) 450 mg of sucrose, and
(iv) 1.5 mg of polysorbate 80.

Alternatively, when expressed as an aqueous solution comprising the antibody-drug conjugate at the concentration of 20 mg/mL, the above-described pharmaceutical composition can be expressed as a pharmaceutical composition comprising
(i) 20 mg/mL of the antibody-drug conjugate,
(ii) 25 mM of a histidine buffer,
(iii) 9% of sucrose,
(iv) 0.03% of polysorbate 80, and
(v) water,
wherein the pH of the composition is 5.5.

When the antibody in the antibody-drug conjugate is an anti-HER3 antibody (preferably, an antibody comprising a heavy chain consisting of an amino acid sequence represented by SEQ ID NO: 3 and a light chain consisting of an amino acid sequence represented by SEQ ID NO: 4, or a variant of the antibody in which a lysine residue at the carboxyl terminus of the heavy chain is deleted), the pharmaceutical composition of the present invention is preferably a pharmaceutical composition comprising,
(i) per 20 mg of the antibody-drug conjugate,
(ii) 25 mmol of a histidine buffer,
(iii) 90 mg of sucrose, and
(iv) 0.3 mg of polysorbate 20.

Furthermore, the pH of the pharmaceutical composition when the antibody-drug conjugate is dissolved in water at a concentration of 20 mg/mL is preferably 5.2 to 5.6, more preferably 5.3 to 5.5, still more preferably 5.4.

When the histidine buffer is expressed by the content of L-histidine and L-histidine hydrochloride, the above-described pharmaceutical composition when the pH thereof is 5.4 can be expressed as a pharmaceutical composition comprising,
(i) per 20 mg of the antibody-drug conjugate,
(ii) 0.81 mg of L-histidine and 4.14 mg of L-histidine hydrochloride hydrate,
(iii) 90 mg of sucrose, and
(iv) 0.3 mg of polysorbate 20.

Furthermore, when expressed as a unit formulation comprising 100 mg of the antibody-drug conjugate, the above-described pharmaceutical composition when the pH thereof is 5.4 can be expressed as a pharmaceutical composition comprising
(i) 100 mg of the antibody-drug conjugate,
(ii) 4.06 mg of L-histidine and 20.7 mg of L-histidine hydrochloride hydrate,
(iii) 450 mg of sucrose, and
(iv) 1.5 mg of polysorbate 20.

Alternatively, when expressed as an aqueous solution comprising the antibody-drug conjugate at the concentration of 20 mg/mL, the above-described pharmaceutical composition can be expressed as a pharmaceutical composition comprising
(i) 20 mg/mL of the antibody-drug conjugate,
(ii) 25 mM of a histidine buffer,
(iii) 9% of sucrose,
(iv) 0.03% of polysorbate 20, and
(v) water,
wherein the pH of the composition is 5.4.

When the antibody in the antibody-drug conjugate is an anti-TROP2 antibody (preferably, an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6, or a variant of the antibody in which a lysine residue at the carboxyl terminus of the heavy chain is deleted), the pharmaceutical composition of the present invention is preferably,
a pharmaceutical composition comprising,
(i) per 20 mg of the antibody-drug conjugate,
(ii) 10 mmol of the histidine buffer,
(iii) 90 mg of sucrose, and
(iv) 0.2 or 0.3 mg of polysorbate 80.

Furthermore, the pH of the pharmaceutical composition when the antibody-drug conjugate is dissolved in water at a concentration of 20 mg/mL is preferably 5.8 to 6.2, more preferably 5.9 to 6.1, still more preferably 6.0.

When the histidine buffer is expressed by the content of L-histidine and L-histidine hydrochloride, the above-described pharmaceutical composition when the pH thereof is 6.0 can be expressed preferably as a pharmaceutical composition comprising,
(i) per 20 mg of the antibody-drug conjugate,
(ii) 0.78 mg of L-histidine and 1.05 mg of L-histidine hydrochloride hydrate,
(iii) 90 mg of sucrose, and
(iv) 0.2 or 0.3 mg of polysorbate 80.

When expressed as a unit formulation comprising 100 mg of the antibody-drug conjugate, the above-described pharmaceutical composition when the pH thereof is 6.0 can be expressed as a pharmaceutical composition comprising
(i) 100 mg of the antibody-drug conjugate,
(ii) 3.88 mg of L-histidine and 5.26 mg of L-histidine hydrochloride hydrate,
(iii) 450 mg of sucrose, and
(iv) 1.0 or 1.5 mg of polysorbate 80.

Alternatively, when expressed as an aqueous solution comprising the antibody-drug conjugate at the concentration of 20 mg/mL, the above-described pharmaceutical composition can be expressed as a pharmaceutical composition comprising
(i) 20 mg/mL of the antibody-drug conjugate,
(ii) 10 mM of the histidine buffer,
(iii) 9% of sucrose,
(iv) 0.02 or 0.03 % of polysorbate 80, and
(v) water,
wherein the pH of the composition is 6.0.

When the antibody in the antibody-drug conjugate is an anti-B7-H3 antibody (preferably, wherein the antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which a lysine residue at the carboxyl terminus of the heavy chain is deleted), the pharmaceutical composition of the present invention is preferably a pharmaceutical composition comprising,
(i) per 20 mg of the antibody-drug conjugate,
(ii) 10 mmol of a histidine buffer,
(iii) 90 mg of sucrose, and
(iv) 0.2 or 0.3 mg of polysorbate 20.

Furthermore, the pH of the pharmaceutical composition when the antibody-drug conjugate is dissolved in water at a concentration of 20 mg/mL is preferably 5.7 to 6.1, more preferably 5.8 to 6.0, still more preferably 5.9.

When the histidine buffer is expressed by the content of L-histidine and L-histidine hydrochloride, the above-described pharmaceutical composition when the pH thereof is 5.9 can be expressed as a pharmaceutical composition comprising,
(i) per 20 mg of the antibody-drug conjugate,
(ii) 0.65 mg of L-histidine and 1.22 mg of L-histidine hydrochloride hydrate,
(iii) 90 mg of sucrose, and
(iv) 0.2 or 0.3 mg of polysorbate 20.

Furthermore, when expressed as a unit formulation comprising 100 mg of the antibody-drug conjugate, the above-described pharmaceutical composition when the pH thereof is 5.9 can be expressed as a pharmaceutical composition comprising
(i) 100 mg of the antibody-drug conjugate,
(ii) 3.23 mg of L-histidine and 6.12 mg of L-histidine hydrochloride hydrate,
(iii) 450 mg of sucrose, and
(iv) 1.0 or 1.5 mg of polysorbate 20.

Alternatively, when expressed as an aqueous solution comprising the antibody-drug conjugate at the concentration of 20 mg/mL, the above-described pharmaceutical composition can be expressed as a pharmaceutical composition comprising
(i) 20 mg/mL of the antibody-drug conjugate,
(ii) 10 mM of a histidine buffer,
(iii) 9% of sucrose, and
(iv) 0.02 or 0.03 % of polysorbate 20, and
(v) water,
wherein the pH of the composition is 5.9.

When the antibody in the antibody-drug conjugate is an anti-GPR20 antibody (preferably, an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10, or a variant of the antibody in which a lysine residue at the carboxyl terminus of the heavy chain is deleted, the pharmaceutical composition of the present invention is, preferably, comprising,
(i) per 20 mg of the antibody-drug conjugate,
(ii) 10 mmol of a histidine buffer,
(iii) 90 mg of sucrose, and
(iv) 0.3 mg of polysorbate 80.

Furthermore, the pH of the pharmaceutical composition when the antibody-drug conjugate is dissolved in water at a concentration of 20 mg/mL is preferably 5.2 to 5.6, more preferably 5.3 to 5.5, still more preferably 5.4.

When the histidine buffer is expressed by the content of L-histidine and L-histidine hydrochloride, the above-described pharmaceutical composition when the pH thereof is 5.4 can be expressed, preferably, as a pharmaceutical composition comprising,
(i) per 20 mg of the antibody-drug conjugate,
(ii) 0.32 mg of L-histidine and 1.66 mg of L-histidine hydrochloride hydrate,
(iii) 90 mg of sucrose, and
(iv) 0.3 mg of polysorbate 80.

Furthermore, when expressed as a unit formulation comprising 100 mg of the antibody-drug conjugate, the above-described pharmaceutical composition when the pH thereof is 5.4 can be expressed as a pharmaceutical composition comprising
(i) 100 mg of the antibody-drug conjugate,
(ii) 1.62 mg of L-histidine and 8.29 mg of L-histidine hydrochloride hydrate,
(iii) 450 mg of sucrose, and
(iv) 1.5 mg of polysorbate 80.

Alternatively, when expressed as an aqueous solution comprising the antibody-drug conjugate at the concentration of 20 mg/mL, the above-described pharmaceutical composition can be expressed as a pharmaceutical composition comprising
(i) 20 mg/mL of the antibody-drug conjugate,
(ii) 10 mM of the histidine buffer,
(iii) 9% of sucrose, and
(iv) 0.03% of polysorbate 80, and
(v) water,
wherein the pH of the composition is 5.4.

The pharmaceutical composition of the present invention is in the form of preferably an injection, more preferably, an aqueous injection or a lyophilized injection, even more preferably a lyophilized injection.

In the present invention, the term "injection" refers to a pharmaceutical composition for administration with a needle to biological tissues e.g., intravenous, intradermally, subcutaneously, intramuscularly, and intraperitonealy. In the injections of the present invention, not only liquid injections, but also solid injections dissolved at the time of use for use in liquids are included. For the liquid injection, the solvent may be water, or a solvent other than water, or a mixed solvent of water and a solvent other than water.

In the present invention, the term "aqueous injection" refers to a pharmaceutical composition which is in the form of a liquid injection and the solvent of which is water (preferably, water for injection).

In the present invention, the term "lyophilized injection" is a solid injection which may be used by dissolving at the time of use in water (preferably, water for injection), which is obtained by lyophilizing an aqueous solution comprising a predetermined amount of a pharmaceutical component.

When the pharmaceutical composition of the present invention is in the form of an aqueous injection, the concentration of the antibody-drug conjugate is preferably 5 to 60 mg/mL, more preferably 15 to 40 mg/mL, furthermore preferably 20 mg/mL.

When the pharmaceutical composition of the present invention is in the form of an aqueous injection, the pharmaceutical composition of the present invention can be preferably stored in a frozen state.

When the pharmaceutical composition of the present invention is in the form of a lyophilized injection, the pharmaceutical composition of the present invention can be preferably stored housed in a brown vial.

When the pharmaceutical composition of the present invention is in the form of a lyophilized injection, a kit comprising the pharmaceutical composition of the present invention and water for injection can be suitably used. Herein, the water for injection can be preferably stored in a state of being housed in an ampoule.

A lyophilized injection wherein the content of the antibody-drug conjugate used in the present invention is 20 mg can be suitably used by redissolving in 1 mL of water for injection. Furthermore, a lyophilized injection wherein the content of the antibody-drug conjugate used in the present invention is 100 mg can be suitably used by redissolving in 5 mL of water for injection.

When the pharmaceutical composition of the present invention is in the form of a lyophilized injection, the method for producing a pharmaceutical composition of the present invention, preferably, comprises the steps of
(1) preparing an aqueous solution comprising predetermined amounts of
   (i) an antibody-drug conjugate,
   (ii) a histidine buffer,
   (iii) sucrose or trehalose, and
   (iv) a surfactant,
(2) if necessary, adjusting the pH of the aqueous solution to a predetermined value, and then,
(3) lyophilizing the aqueous solution.

The step of lyophilizing the aqueous solution preferably comprises a process of annealing at a shelf temperature near the eutectic point of the aqueous solution. Herein "near the eutectic point" indicates the range of from the temperature which is 1.5°C lower than the eutectic point to the temperature which is 1.5°C higher than the eutectic point.

In the present invention, the term "annealing" means a process of growing ice crystals in the frozen body at a product temperature equal to or more than the freezing glass transition point (Tg'). As ice crystals get larger, passages of water (water vapor) that sublimes at drying become larger. This reduces the sublimation resistance, and can be expected to improve the form of the lyophilized cake and shorten the drying time.

In the present invention, the term "shelf temperature" means the temperature in the apparatus (in the system) for performing lyophilization.

In the present invention, the term "product temperature" means the temperature of the object (product) to be lyophilized.

In the present invention, the term "lyophilized cake" means the lyophilized body (solid having a porous structure) obtained by the series of lyophilization processes.

Annealing, as described above, can be suitably performed from a temperature 1.5°C lower than the eutectic point of the aqueous solution to a temperature 1.5°C higher than the eutectic point, more preferably can be performed at a shelf temperature which is same as the eutectic point of the aqueous solution.

These production methods are preferably characterized in that the time for the process of primary drying is shortened compared with that when annealing at a shelf temperature which is 5°C lower than the eutectic point is performed. Furthermore, these production methods are preferably characterized in that a lyophilized cake having less shrinkage is obtained compared with that when annealing at a shelf temperature which is 5°C lower than the eutectic point is performed.

Since the eutectic point of the pharmaceutical composition of the present invention is about -3°C to about -1°C, the annealing can be carried out preferably at a shelf temperature of -4.5°C to 0.5°C, more preferably at a shelf temperature of -4 to -1°C, even more preferably at a shelf temperature of -4 to -2°C, even more preferably at a shelf temperature of -3 to -2°C, and even more preferably more at a shelf temperature of - 2.5°C.

After annealing, the first drying process and the second drying process are carried out, then the pharmaceutical composition of the present invention is produced in the form of a lyophilized injection.

In the present invention, the term "primary drying process" is one of the production processes of the lyophilized injection, and it means a process for sublimating free water in the frozen body.

In the present invention, the term "secondary drying process" in the present invention is one of the production processes of the lyophilized injection, and it means a process for sublimating water bound to the solute (bound water).

The primary drying process can be carried out preferably at a shelf temperature of -5 to 5°C under a vacuum of 5 to 15 Pa, more preferably at a shelf temperature of -4 to 4°C under a vacuum of 7 to 13 Pa, even more preferably at a shelf temperature of -3 to 3°C under a vacuum of 8 to 12 Pa, even more preferably at a shelf temperature of -2 to 2°C under a vacuum of 9 to 11 Pa, even more preferably at a shelf temperature of -1 to 1°C under a vacuum of 9 to 11 Pa, and even more preferably at a shelf temperature of 0°C under a vacuum of about 10 Pa.

The secondary drying process can be carried out preferably at a shelf temperature of 40 to 50°C under a vacuum of 5 to 15 Pa or without vacuum, more preferably at a shelf temperature of 42 to 48°C under a vacuum of 7 to 13 Pa, even more preferably at a shelf temperature of 44 to 46°C under a vacuum of 9 to 11 Pa, and even more preferably at a shelf temperature of 45°C under a vacuum of 10 Pa.

The quality of the pharmaceutical compositions of the present invention can be confirmed by evaluating storage stabilities at 1 month, 3 months, 6 months, 12 months, 18 months, 24 months, or 36 months under 50°C, under 40°C/75%RH, under 25°C/60%RH, or under 5°C by employing protein concentration, moisture, insoluble microparticles, DAR, ratios of monomers, aggregates and fragments, NPI, IoP, charge isomers, and pH as indices. Furthermore, depending on the type of antibody in the antibody-drug conjugate, the quality can be evaluated by employing, for example, binding activity or potency (Bioassay) to an antigen as an index. Furthermore, quality evaluation employing drug distribution or rCE-SDS as an index, and quality evaluation by property (appearance) observation can also be performed. When the pharmaceutical composition is in the form of an aqueous injection, the quality can be evaluated by employing osmotic pressure ratio as an index, and when the pharmaceutical composition is in the form of a lyophilized injection, the quality can be evaluated by employing redissolution time as an index. With these quality evaluations, it is also possible to confirm the superiority of the present pharmaceutical composition to existing pharmaceutical compositions.

The lyophilization method of the present invention is expected to have general applicability, not only to the pharmaceutical composition comprising the antibody-drug conjugate used in the present invention, but also to an aqueous solution containing sucrose or trehalose.

Thus, a method for producing a lyophilized injection comprising a process of annealing an aqueous solution containing sucrose or trehalose at a shelf temperature near the eutectic point of the aqueous solution, wherein near the eutectic point indicates the range of from a temperature which is 1.5°C lower than the eutectic point to a temperature which is 1.5°C higher than the eutectic point is also included within the scope of the present invention.

Furthermore, the annealing may suitably be carried out at a shelf temperature same as the eutectic point of the aqueous solutions containing sucrose or trehalose.

These production methods are preferably characterized in that the time for the process of primary drying is shortened compared with that when annealing at a shelf temperature which is 5°C lower than the eutectic point is performed.

Furthermore, these production methods are preferably characterized in that a lyophilized cake having less shrinkage is obtained compared with that when annealing at a shelf temperature which is 5°C lower than the eutectic point is performed.

### [Use of pharmaceutical composition]

The pharmaceutical composition of the present invention can be expected to exert a therapeutic effect by application as a systemic therapy to patients, and additionally, by local application to cancer tissues.

The pharmaceutical composition of the present invention can be preferably used for a mammal, but is more preferably used for a human.

Examples of the administration route which may be used to administer the pharmaceutical compositions of the present invention include intravenous, intradermal, subcutaneous, intramuscular, and intraperitoneal routes, but intravenous administration is a preferred administration route.

When the pharmaceutical compositions of the present invention is in the form of an aqueous injection, the aqueous injection can be preferably infused intravenously after diluted with a suitable diluent. Examples of the diluent include glucose solution (preferably, 5% glucose solution) and physiological saline.

When the pharmaceutical composition of the present invention is in the form of a lyophilized injection, the lyophilized injection can be dissolved by water (preferably, water for injection), and infused intravenously after dilution with a suitable diluent. Examples of the diluent include glucose solution (preferably, 5% glucose solution) and physiological saline.

The pharmaceutical composition of the present invention can exhibit a pharmaceutical effect even at a small dosage when the antibody-drug conjugate has a higher affinity for an antigen, that is, in terms of the dissociation constant (Kd value), a higher affinity (lower Kd value) for the antigen for the antibody-drug conjugate of the present invention. Thus, the dosage of the pharmaceutical composition of the present invention can be determined in view of the situation relating to the affinity with the antigen. When the pharmaceutical composition of the present invention is administered to a human, for example, about 0.001 to 100 mg/kg as an antibody-drug conjugate, wherein the "mg/kg" means the dose of the antibody-drug conjugate per 1 kg of human body weight, may be administered once or administered in several portions with intervals of 1 to 180 days. For example, when the antibody-drug conjugate used in the present invention is an anti-HER2 antibody-drug conjugate, examples of the administration method of the pharmaceutical composition of the present invention include an administering method of 0.8 mg/kg to 8 mg/kg once every three weeks, more preferably, 0.8 mg/kg, 1.6 mg/kg, 3.2 mg/kg, 5.4 mg/kg, 6.4 mg/kg, 7.4 mg/kg, or 8 mg/kg once every three weeks, even more preferably, 5.4 mg/kg, 6.4 mg/kg, 7.4 mg/kg, or 8 mg/kg once every three weeks, and even more preferably, 5.4 mg/kg, 6.4 mg/kg, 7.4 mg/kg, or 8 mg/kg once every three weeks, and even more preferably, 5.4 mg/kg and 6.4 mg/kg once every three weeks.

The pharmaceutical composition of the present invention can be used for the treatment of cancer, preferably, can be used for the treatment of at least one cancer selected from the group consisting of breast cancer, gastric cancer, (also called gastric adenocarcinoma), colorectal cancer (also called colon and rectal cancer, and including colon cancer and rectal cancer), lung cancer (including small cell lung cancer and non-small cell lung cancer), esophageal cancer, salivary gland cancer, gastroesophageal junction adenocarcinoma, bile duct cancer, Paget disease, pancreatic cancer, ovarian cancer, uterine cancer sarcoma, urothelial cancer, prostate cancer, bladder cancer, gastrointestinal stromal tumor, gastrointestinal stromal tumor, cervical cancer, squamous cell carcinoma, peritoneal cancer, liver cancer, hepatocellular carcinoma, colon cancer, rectal cancer, endometrial cancer, uterine cancer, kidney cancer, vulval cancer, thyroid cancer, penile cancer, leukemia, malignant lymphoma, plasmacytoma, myeloma, nerve epithelial tissue tumors, nerve sheath tumors, head and neck cancer, skin cancer, throat cancer, gallbladder, bile duct cancer, mesothelioma, and sarcoma. For example, when the antibody-drug conjugates used in the present invention is an anti-ER2 antibody-drug conjugate, the pharmaceutical compositions of the present invention more preferably can be used for the treatment of at least one cancer selected from the group consisting of breast cancer, gastric cancer, colorectal cancer, non-small cell lung cancer, esophageal cancer, salivary gland cancer, esophagogastric junction adenocarcinoma, bile duct cancer, Paget disease, pancreatic cancer, ovarian cancer, and uterine cancer sarcoma; more preferably can be used for the treatment of at least one cancer selected from the group consisting of breast cancer, gastric cancer, colorectal cancer, non-small cell lung cancer, esophageal cancer, salivary gland cancer, esophagogastric junction adenocarcinoma, bile duct cancer, and Paget disease; and even more preferably can be used for the treatment of breast cancer, gastric cancer, colorectal cancer or non-small cell lung cancer.

The pharmaceutical composition of the present invention can be selectively used as an agent for drug therapy, which is a main method for treating cancer, and as a result, can delay development of cancer cells, inhibit growth thereof, and further kill cancer cells. These effects can allow cancer patients to be free from symptoms caused by cancer or achieve improvement in QOL of cancer patients and attain a therapeutic effect by sustaining the lives of the cancer patients. Even if the pharmaceutical composition and therapeutic method of the present invention do not accomplish killing cancer cells, they can achieve higher QOL of cancer patients while achieving longer-term survival, by inhibiting or controlling the growth of cancer cells.

In such a drug therapy, the pharmaceutical composition of the present invention can be used alone, and in addition, they can be used in combination with an additional therapy in adjuvant therapy and can be combined with surgery, radiotherapy, hormone therapy, or the like. Furthermore, they can also be used for drug therapy in neoadjuvant therapy.

In addition to the therapeutic use as described above, for example, a prophylactic effect such as suppressing the growth of small metastatic cancer cells and further killing them can also be expected for the pharmaceutical composition according to the present invention. For example, an effect of inhibiting and killing cancer cells in a body fluid in the course of metastasis or an effect of, for example, inhibiting and killing small cancer cells immediately after implantation in any tissue can be expected. Accordingly, inhibition of cancer metastasis or a prophylactic effect can be expected, particularly, after surgical removal of cancer.

The pharmaceutical composition of the present invention can be administered in combination with other cancer treating agents. The anti-cancer effect may be enhanced accordingly. Examples of the other cancer treating agents used for such purpose include 5-fluorouracil (5-FU), pertuzumab, trastuzumab, paclitaxel, carboplatin, cisplatin, gemcitabine, capecitabine, irinotecan (CPT-11), docetaxel, pemetrexed, sorafenib, vinblastin, vinorelbine, everolims, tanespimycin, bevacizumab, oxaliplatin, lapatinib, trastuzumab emtansine (T-DM1) or agents described in International Publication No. WO 2003/038043, LH-RH analogues (leuprorelin, goserelin, or the like), estramustine phosphate, estrogen antagonists (tamoxifen, raloxifene, or the like), and aromatase inhibitors (anastrozole, letrozole, exemestane, or the like), but are not limited as long as they are agents having an antitumor activity. Examples

The present invention is specifically described in view of the examples shown below. However, the present invention is not limited to these. Further, it is by no means to be interpreted in a limited way.

### [Example 1: Production of antibody-drug conjugate (1)]

In accordance with a production method described in International Publication No. WO 2015/115091 with use of a humanized anti-HER2 antibody (trastuzumab), an antibody-drug conjugate in which a drug-linker represented by the following formula: 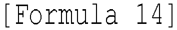 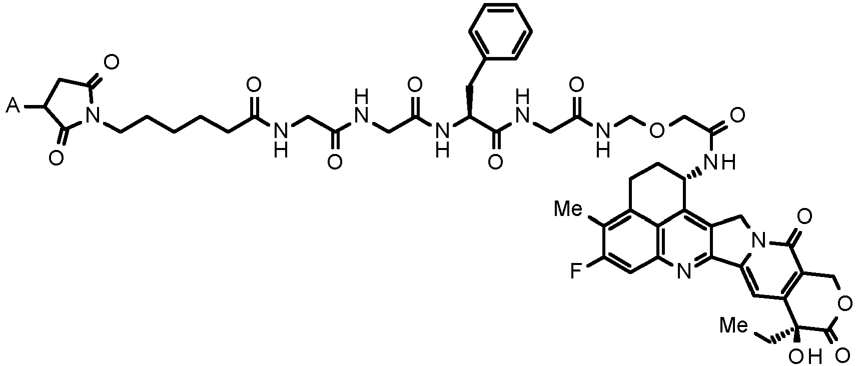 wherein A represents a connecting position to an antibody, is conjugated to the antibody via a thioether bond (hereinafter, referred to as antibody-drug conjugate (1)), was produced. The DAR of antibody-drug conjugate (1) was 7.8.

### [Example 2: Preparation of formulation of antibody-drug conjugate (1)]

### Preparation Method of Formulation (1)

An aqueous solution (pH 5.8) containing antibody-drug conjugate (1) (20 mg/mL), 10 mM of a histidine buffer, 11.1% of trehalose hydrate, and 0.02% of polysorbate 20 was dispensed into a dialysis cassette (dialysis membrane, Slide-A-lyzer, MWCO 20,000), and dialyzed with a buffer containing excipient of purpose formulation at 50 times or more of the sample volume injected into the dialysis cassette. The dialysis was performed twice at 5°C for 5 hours or more. After the dialysis, concentration or dilution was appropriately carried out so that the protein concentration became about 20 mg/mL. This solution was filtered through a 0.22 µm filter. The filtered solution was filled into glass vials by 1 mL, the vials were half stoppered with rubber plugs, and lyophilization was carried out under the conditions shown in Table 1. After lyophilization, the vials stoppered with the rubber plugs were sealed with caps. Filtration and filling operations were carried out within a clean bench.

**[Table 1]**

| Process | Step No. | Shelf temperature (°C) | Time (min) | Chamber vacuum degree (Pa) |
|---|---|---|---|---|
| Freezing | 1 | 5 | 30 | Without vacuum |
| | 2 | 5 | 120 | |
| | 3 | -40 | 60 | |
| | 4 | -40 | 120 | |
| | 5 | -5 | 60 | |
| | 6 | -5 | 120 | |
| | 7 | -40 | 60 | |
| | 8 | -40 | 120 | |
| Primary drying | 9 | -20 | 60 | 10 |
| | 10 | -20 | 1200 to 1800 | |
| Secondary drying | 11 | 30 | 120 | Without vacuum control |
| | 12 | 30 | 900 to 1800 | |
| | 13 | 5 | 60 | |

### Preparation Method of Formulation (2)

An aqueous solution (pH 5.8) containing antibody-drug conjugate (1) (20 mg/mL), 10 mM of a histidine buffer, 11.1% of trehalose hydrate, and 0.02% of polysorbate 20 was dispensed into a UF membrane kit (AMICON ULTRA-15, 30kDa) and centrifuged at a setting of 3000 rpm and 5°C to be concentrated to about 2 times. Polysorbate 20 contained in the concentrated solution was removed by adsorbing on a hydrophobic adsorbent (Amberlite XAD7HP). The obtained chemical solution after the removal of polysorbate 20 was injected into a dialysis cassette (dialysis membrane, Slide-A-lyzer, MWCO 20,000), and dialyzed with a buffer containing excipient of purpose formulation at 50 times or more of the sample volume injected into the dialysis cassette. The dialysis was performed twice at 5°C for 5 hours or more. After the dialysis, concentration or dilution was appropriately carried out so that the protein concentration became about 20 mg/mL, and then polysorbate 20 or polysorbate 80 was added to the resultant solution and mixed. This solution was filtered through a 0.22 µm filter. The filtered solution was filled into glass vials by 1 mL, the vials were half stoppered with rubber plugs, and lyophilization was carried out under the conditions shown in Table 2. After lyophilization, the vials stoppered with the rubber plugs were sealed with caps. Filtration and filling operations were carried out within a clean bench.

**[Table 2]**

| Process | Step No. | Shelf temperature (°C) | Time (min) | Chamber vacuum degree (Pa) |
|---|---|---|---|---|
| Freezing | 1 | 5 | 30 | Without vacuum |
| | 2 | 5 | 120 | |
| | 3 | -40 | 60 | |
| | 4 | -40 | 120 | |
| | 5 | -7 | 60 | |
| | 6 | -7 | 120 | |
| | 7 | -40 | 60 | |
| | 8 | -40 | 120 | |
| Primary drying | 9 | -20 | 60 | Without vacuum control |
| | 10 | -20 | 2400 | |
| Secondary drying | 11 | 40 | 120 | |
| | 12 | 40 | 900 | |
| | 13 | 5 | 60 | |

### [Example 3: Stability Test]

Lyophilized injections were stored at 40°C/75% RH for 3 months. Their stabilities were compared and evaluated by employing protein concentration (measured by UV method), moisture (measured by Karl Fischer method), insoluble microparticles (measured by Micro Flow Imaging), Drug Antibody Ratio (DAR) (measured by HPLC method), ratios of monomers, aggregates, and fragments (measured by SE-HPLC (SEC)), Non Proteinous Impurity (NPI) (measured by HPLC method), Impurity of Payload (IoP) (measured by HPLC method), charge isomers (measured by CZE), pH, and HER2 binding activity (measured by ELISA method) as indices.

### [Example 4: Excipient Screening]

The excipient of lyophilized injections was selected from 10% trehalose hydrate, 9% sucrose, and 5% sorbitol, which have been actually used in biopharmaceuticals and in these concentrations make the osmotic pressure substantially isotonic. Other components in the lyophilized injections were commonly set to antibody-drug conjugate (1) (20 mg/mL), 40 mM of a histidine buffer, and 0.02% of polysorbate 20, and the pH of the formulations was set to 5.8.

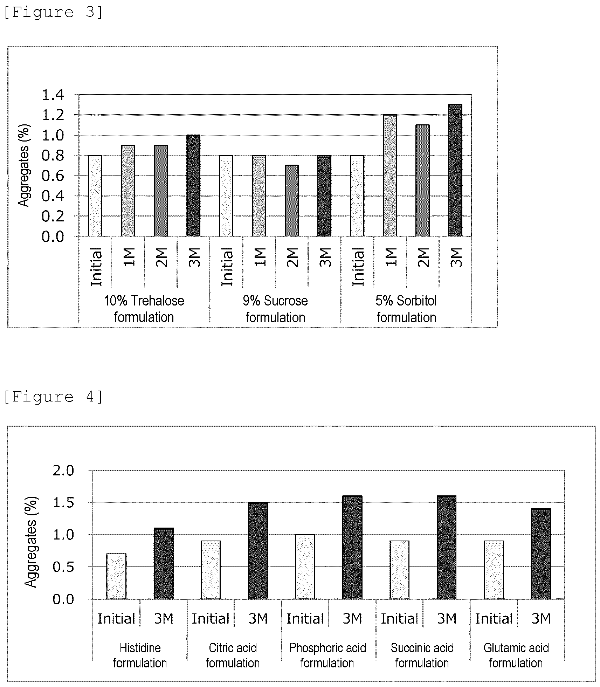

The results are shown in Table 3. It was found that the generation of aggregates was smaller when using 9% sucrose or 10% trehalose hydrate compared with when using 5% of sorbitol. In particular, it was found that the generation of aggregates becomes smallest when using 9% sucrose (Figure 3).

**[Table 3]**

| Test items | | 10% Trehalose formulation | | | | 9% Sucrose formulation | | | | 5% Sorbitol formulation | | | |
|---|---|---|---|---|---|---|---|---|---|---|---|---|---|
| | | 40°C/75%RH | | | | 40°C/75%RH | | | | 40°C/75%RH | | | |
| | | Initial | 1M | 2M | 3M | Initial | 1M | 2M | 3M | Initial | 1M | 2M | 3M |
| Protein content (mg/mL) | | 21.3 | 21.9 | 20.6 | 17.9 | 21.9 | 22.0 | 19.9 | 20.1 | 23.1 | 22.8 | 21.6 | 20.9 |
| Moisture (%) | | 0.35 | 0.64 | 0.77 | 1.28 | 0.40 | 0.61 | 1.39 | 1.33 | 0.22 | 0.44 | 1.49 | 1.08 |
| Insoluble microparticles (particles/mL) | ≥10 µm | 19 | 5 | 83 | 86 | 26 | 17 | 167 | 12 | N.A. | 63 | 44 | 31 |
| | ≥25 µm | 2 | 0 | 2 | 12 | 2 | 0 | 28 | 0 | N.A. | 5 | 10 | 0 |
| DAR | | 7.8 | 7.7 | 7.7 | 7.8 | 7.8 | 7.8 | 7.7 | 7.8 | 7.8 | 7.8 | 7.7 | 7.8 |
| SEC (%) | Monomers | 97.3 | 97.3 | 98.0 | 98.1 | 97.4 | 97.5 | 98.2 | 98.2 | 97.5 | 96.9 | 97.9 | 97.7 |
| | Aggregates | 0.8 | 0.9 | 0.9 | 1.0 | 0.8 | 0.8 | 0.7 | 0.8 | 0.8 | 1.2 | 1.1 | 1.3 |
| | Fragments | 1.9 | 1.7 | 1.1 | 1.0 | 1.7 | 1.7 | 1.0 | 1.0 | 1.6 | 1.9 | 1.0 | 1.0 |
| NPI (%) | | 0.65 | 0.69 | 0.70 | 0.66 | 0.64 | 0.66 | 0.66 | 0.57 | 0.60 | 0.60 | 0.66 | 0.61 |
| IoP(%) | | 1.02 | 1.02 | 0.99 | 1.29 | 0.92 | 0.99 | 0.85 | 1.71 | 1.00 | 2.84 | 1.10 | 1.88 |
| Charge isomers (main peak %) | | 54.5 | N.A. | N.A. | 56.7 | 53.4 | N.A. | N.A. | 56.7 | 51.1 | N.A. | N.A. | 51.3 |

### [Example 5: Buffer Screening]

The buffer of lyophilized injections was selected from histidine, citric acid, phosphoric acid, succinic acid, and glutamic acid, which have been used in biopharmaceuticals and have buffering capacities at near pH 4 to pH 7. The concentration of these buffers was uniformly 25 mM. Other components in the lyophilized injections were commonly set to antibody-drug conjugates (1) (20 mg/mL), 10% of trehalose hydrate and 0.02% of polysorbate 20, and the pH of the formulations was set to 5.8.

The results are shown in Table 4. It was found that the generation of aggregates becomes smallest when using histidine as the buffer (Figure 4).

**[Table 4]**

| Test items | | Histidine formulation | | Citric acid formulation | | Phosphoric acid formulation | | Succinic acid formulation | | Glutamic acid formulation | |
|---|---|---|---|---|---|---|---|---|---|---|---|
| | | 40°C/75%RH | | 40°C/75%RH | | 40°C/75%RH | | 40°C/75%RH | | 40°C/75%RH | |
| | | Initial | 3M | Initial | 3M | Initial | 3M | Initial | 3M | Initial | 3M |
| Protein content (mg/mL) | | 19.6 | 19.0 | 22.9 | 20.8 | 22.4 | 20.5 | 19.6 | 20.0 | 22.7 | 19.9 |
| Moisture (%) | | 0.26 | 1.12 | 0.43 | 1.19 | 0.45 | 1.31 | 0.37 | 1.30 | 0.47 | 1.15 |
| Insoluble microparticles (particles/mL) | ≥10 µm | 10 | 19 | 5 | 24 | 12 | 34 | 10 | 12 | N.A. | 7 |
| | ≥25 µm | 2 | 2 | 0 | 0 | 7 | 7 | 0 | 2 | N.A. | 0 |
| DAR | | 7.8 | 7.7 | 7.8 | 7.8 | 7.8 | 7.8 | 7.7 | 7.8 | 7.8 | 7.8 |
| SEC (%) | Monomers | 97.6 | 98.2 | 97.4 | 97.7 | 97.3 | 97.6 | 97.2 | 97.6 | 97.4 | 97.9 |
| | Aggregates | 0.7 | 1.1 | 0.9 | 1.5 | 1.0 | 1.6 | 0.9 | 1.6 | 0.9 | 1.4 |
| | Fragments | 1.7 | 0.7 | 1.6 | 0.7 | 1.7 | 0.8 | 1.8 | 0.7 | 1.7 | 0.7 |
| NPI (%) | | 0.71 | 0.84 | 0.64 | 0.78 | 0.62 | 0.83 | 0.73 | 0.79 | 0.63 | 0.76 |
| IoP(%) | | 1.10 | 1.00 | 1.54 | 2.36 | 1.15 | 2.29 | 1.27 | 2.96 | 1.35 | 0.94 |
| Charge isomers (main peak %) | | 53.4 | 57.9 | 58.8 | 58.4 | 58.2 | 58.7 | 58.6 | 56.3 | 55.9 | 58.4 |

### [Example 6: pH Screening]

For the pH of reconstituted lyophilized injections, a pH was selected from pH 4 to pH 7 considering the use of histidine buffer that was selected by the Buffer Screening, and biocompatibility and safety such as irritancy in administration by intravenous drip infusion. Other components in the lyophilized injections were commonly set to antibody-drug conjugates (1) (20 mg/mL), 9% of sucrose and 25 mM of a histidine buffer and 0.02% of polysorbate 20. Preparation of the samples was carried out by the Preparation Method of Formulation (1).

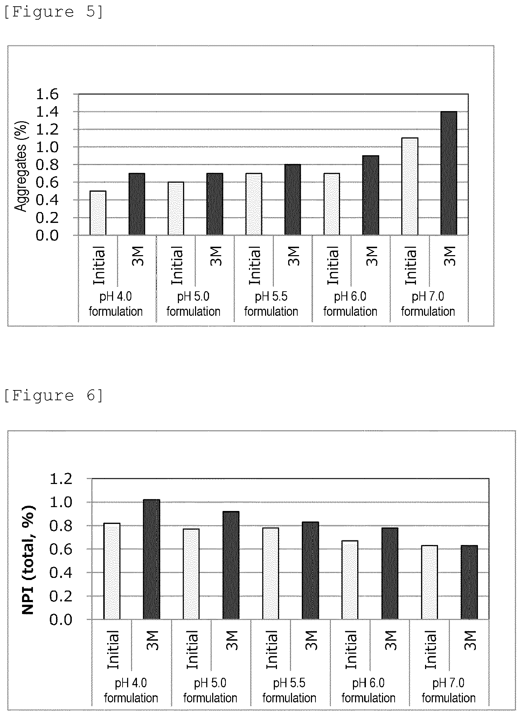

The results are shown in Table 5. It was revealed that as the pH increases, aggregates increase (Figure 5), and as the pH lowers, the NPI increases (Figure 6). Consequently, it was found that it is possible to suppress the increase of aggregates and the increase of NPI in good balance when the pH is 5.5.

**[Table 5]**

| Test items | | pH 4.0 formulation | | pH 5.0 formulation | | pH 5.5 formulation | | pH 6.0 formulation | | pH 7.0 formulation | |
|---|---|---|---|---|---|---|---|---|---|---|---|
| | | 40°C/75%RH | | 40°C/75%RH | | 40°C/75%RH | | 40°C/75%RH | | 40°C/75%RH | |
| | | Initial | 3M | Initial | 3M | Initial | 3M | Initial | 3M | Initial | 3M |
| Protein content (mg/mL) | | 18.1 | 18.4 | 18.2 | 19.0 | 18.7 | 19.1 | 18.4 | 19.1 | 18.9 | 19.2 |
| Moisture (%) | | 0.64 | 1.35 | 0.41 | 1.26 | 0.54 | 1.28 | N.D. | 1.29 | 0.63 | 1.29 |
| Insoluble microparticles (particles/mL) | ≥10 µm | 36 | 7 | 0 | 10 | 17 | 7 | 38 | 14 | 19 | 15 |
| | ≥25 µm | 2 | 0 | 0 | 2 | 0 | 0 | 5 | 0 | 2 | 0 |
| DAR | | 7.7 | 7.7 | 7.7 | 7.7 | 7.8 | 7.7 | 7.7 | 7.7 | 7.7 | 7.7 |
| SEC (%) | Monomers | 99.0 | 98.8 | 98.9 | 98.7 | 98.8 | 98.7 | 98.8 | 98.6 | 98.5 | 98.0 |
| | Aggregates | 0.5 | 0.7 | 0.6 | 0.7 | 0.7 | 0.8 | 0.7 | 0.9 | 1.1 | 1.4 |
| | Fragments | 0.5 | 0.5 | 0.4 | 0.5 | 0.5 | 0.5 | 0.5 | 0.5 | 0.4 | 0.5 |
| NPI (%) | | 0.82 | 1.02 | 0.77 | 0.92 | 0.78 | 0.83 | 0.67 | 0.78 | 0.63 | 0.63 |
| IoP(%) | | 1.18 | 0.77 | 0.74 | 0.82 | 0.84 | 0.96 | 0.94 | 1.27 | 0.90 | 2.12 |
| Charge isomers (main peak %) | | 61.6 | 62.9 | 62.4 | 62.9 | 60.4 | 62.6 | 59.1 | 61.0 | 51.5 | 57.1 |

### [Example 7: Screening on Concentration of Buffer]

The concentration of the histidine buffer, which was selected as the buffer of lyophilized injections, was selected from the range of 10 mM to 40 mM. Other components in the lyophilized injections were commonly set to antibody-drug conjugates (1) (20 mg/mL), 9% of sucrose and 0.02% of polysorbate 20, and the pH of the formulations was 5.5. Preparation of the samples was carried out according to the Preparation Method of Formulation (1).

The results are shown in Table 6. Any of the samples showed comparable stabilities when the concentration of the histidine buffer was 10 mM to 40 mM.

**[Table 6]**

| Test items | | Concentration of histidine buffer | | | | | |
|---|---|---|---|---|---|---|---|
| | | 10mM | | 25mM | | 40mM | |
| | | 40°C/75%RH | | 40°C/75%RH | | 40°C/75%RH | |
| | | Initial | 3M | Initial | 3M | Initial | 3M |
| Protein content (mg/mL) | | 19.2 | 19.2 | 18.7 | 19.1 | 18.5 | 19.0 |
| pH | | 5.59 | 5.55 | 5.52 | 5.47 | 5.56 | 5.51 |
| Moisture (%) | | 0.44 | 1.27 | 0.54 | 1.28 | 0.50 | 1.28 |
| Insoluble microparticles (particles/mL) | ≥10 µm | 10 | 19 | 17 | 7 | 14 | 17 |
| | ≥25 µm | 0 | 0 | 0 | 0 | 0 | 5 |
| DAR | | 7.7 | 7.7 | 7.8 | 7.7 | 7.8 | 7.7 |
| SEC (%) | Monomers | 98.9 | 98.7 | 98.8 | 98.7 | 98.8 | 98.7 |
| | Aggregates | 0.6 | 0.8 | 0.7 | 0.8 | 0.7 | 0.8 |
| | Fragments | 0.5 | 0.5 | 0.5 | 0.5 | 0.5 | 0.5 |
| NPI (%) | | 0.72 | 0.84 | 0.78 | 0.83 | 0.73 | 0.86 |
| IoP(%) | | 0.81 | 1.01 | 0.84 | 0.96 | 0.90 | 0.98 |
| Charge isomers (main peak %) | | 59.6 | 61.6 | 60.4 | 62.6 | 60.4 | 61.9 |

### [Example 8: Surfactant Screening]

In lyophilized injections, a surfactant is expected to serve as an interface protective agent of the protein concentration layer during freezing or as an interface protective agent against micro bubbles generated during redissolution of the lyophilized body. Considering this, 0.02% to 0.04% of polysorbate 20 (Tween 20) and polysorbate 80 (Tween 80) were studied as surfactants. Other components in the lyophilized injections were commonly set to antibody-drug conjugates (1) (20 mg/mL), 9% of sucrose and 25 mM of a histidine buffer, and the pH of the formulations was set to 5.5. Preparation of the samples was carried out according to the Preparation Method of Formulation (2).

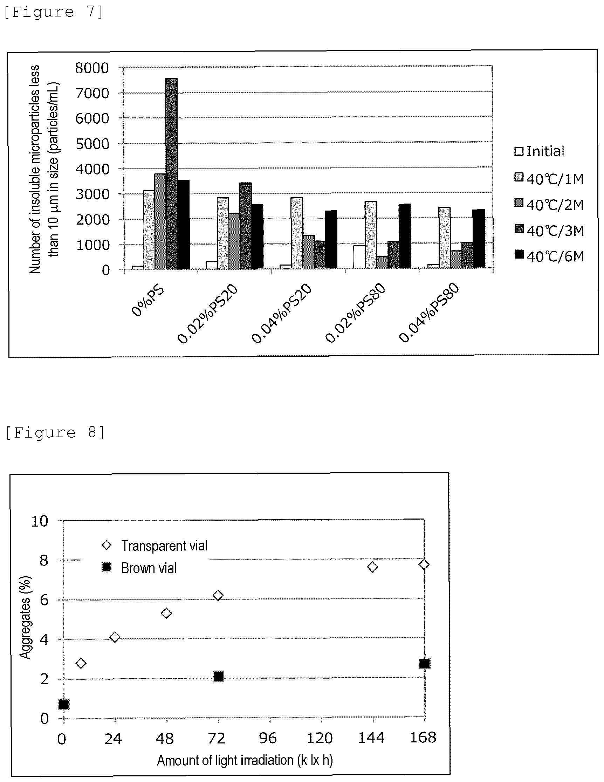

The results are shown in Tables 7 and 8 (wherein, "PS" means polysorbate, "PS20" means polysorbate 20, and "PS80" means polysorbate 80. Suppression of the number of fine particles was observed by the use of 0.02% to 0.04% of polysorbate 20 or 80, and it was found that the use of 0.02% to 0.04% of polysorbate 80 particularly suppresses the number of microparticles (Figure 7).

**[Table 7]**

| Test items | | 0% PS | | | | 0.02% PS20 | | | | 0.04% PS20 | | | |
|---|---|---|---|---|---|---|---|---|---|---|---|---|---|
| | | 40°C/75%RH | | | | 40°C/75%RH | | | | 40°C/75%RH | | | |
| | | Initial | 1M | 2M | 3M | Initial | IM | 2M | 3M | Initial | 1M | 2M | 3M |
| Protein content (mg/mL) | | 17.8 | 18.5 | 18.3 | 17.9 | 18.2 | 18.6 | 18.6 | 18.4 | 18.8 | 18.9 | 19.1 | 18.8 |
| pH | | 5.6 | 5.6 | 5.5 | 5.5 | 5.6 | 5.6 | 5.5 | 5.5 | 5.6 | 5.5 | 5.5 | 5.5 |
| Moisture (%) | | 0.4 | 0.6 | 0.9 | 1.1 | 0.3 | 0.6 | 0.8 | 1.1 | 0.4 | 0.7 | 0.9 | 1.1 |
| Insoluble microparticles (particles/mL) | <10 µm | 132 | 3120 | 3788 | 7570 | 314 | 2832 | 2214 | 3419 | 154 | 2810 | 1334 | 1107 |
| | ≥10 µm | 0 | 81 | 138 | 72 | 2 | 0 | 83 | 61 | 2 | 16 | 89 | 44 |
| | ≥25 µm | 0 | 22 | 28 | 0 | 0 | 0 | 6 | 11 | 0 | 0 | 11 | 17 |
| DAR | | 7.7 | 7.7 | 7.6 | 7.7 | 7.7 | 7.7 | 7.6 | 7.6 | 7.7 | 7.7 | 7.6 | 7.7 |
| SEC (%) | Monomers | 98.8 | 98.8 | 98.8 | 98.8 | 98.8 | 98.7 | 98.7 | 98.8 | 98.8 | 98.8 | 98.8 | 98.8 |
| | Aggregates | 0.7 | 0.8 | 0.8 | 0.8 | 0.7 | 0.8 | 0.8 | 0.8 | 0.7 | 0.8 | 0.8 | 0.8 |
| | Fragments | 0.5 | 0.4 | 0.4 | 0.4 | 0.5 | 0.5 | 0.4 | 0.4 | 0.5 | 0.4 | 0.4 | 0.4 |
| NPI (%) | | 0.00 | 0.05 | 0.07 | 0.09 | 0.00 | 0.05 | 0.07 | 0.09 | 0.00 | 0.05 | 0.07 | 0.10 |
| IoP(%) | | 0.78 | 0.79 | 0.94 | 0.83 | 0.78 | 0.79 | 0.98 | 0.81 | 0.79 | 0.83 | 0.99 | 0.82 |
| HER2 binding activity (%) | | 86 | n.t. | n.t. | 83 | 82 | n.t. | n.t. | 78 | n.t. | n.t. | n.t. | n.t. |

**[Table 8]**

| Test items | | 0.02% PS80 | | | | 0.04% PS80 | | | |
|---|---|---|---|---|---|---|---|---|---|
| | | 40°C/75%RH | | | | 40°C/75%RH | | | |
| | | Initial | 1 M | 2M | 3M | Initial | 1M | 2M | 3M |
| Protein content (mg/mL) | | 18.7 | 18.8 | 19.3 | 18.7 | 18.7 | 18.9 | 19.2 | 18.7 |
| pH | | 5.6 | 5.5 | 5.5 | 5.6 | 5.6 | 5.6 | 5.6 | 5.5 |
| Moisture (%) | | 0.4 | 0.6 | 0.9 | 1.2 | 0.4 | 0.8 | 0.9 | 1.1 |
| Insoluble microparticles (particles/mL) | <10 µm | 918 | 2663 | 476 | 1068 | 134 | 2411 | 694 | 1030 |
| | ≥10 µm | 7 | 38 | 28 | 28 | 5 | 22 | 11 | 83 |
| | ≥25 µm | 5 | 0 | 0 | 17 | 0 | 6 | 6 | 33 |
| DAR | | 7.7 | 7.7 | 7.7 | 7.6 | 7.7 | 7.7 | 7.6 | 7.6 |
| SEC (%) | Monomers | 98.8 | 98.8 | 98.8 | 98.8 | 98.8 | 98.8 | 98.8 | 98.8 |
| | Aggregates | 0.7 | 0.8 | 0.8 | 0.8 | 0.7 | 0.8 | 0.8 | 0.8 |
| | Fragments | 0.5 | 0.4 | 0.4 | 0.4 | 0.5 | 0.4 | 0.4 | 0.4 |
| NPI (%) | | 0.00 | 0.05 | 0.07 | 0.09 | 0.00 | 0.05 | 0.07 | 0.09 |
| IoP(%) | | 0.75 | 0.86 | 0.99 | 0.82 | 0.79 | 0.82 | 0.97 | 0.85 |
| HER2 binding activity (%) | | 86 | n.t. | n.t. | 87 | n.t. | n.t. | n.t. | n.t. |

### [Example 9: Study on vial]

In each of brown glass vials and transparent glass vials, lyophilized injections were filled, and quality changes of the formulations upon exposure to light under a white fluorescent lamp of 1,000 lx were compared and studied. The components of the lyophilized injections were commonly set to antibody-drug conjugate (1) (20 mg/mL), 9% of sucrose, 25 mM of a histidine buffer, and 0.02% of polysorbate 20, and the pH of the formulations was set to 5.5. Preparation of the samples was carried out according to the Preparation Method of Formulation (1).

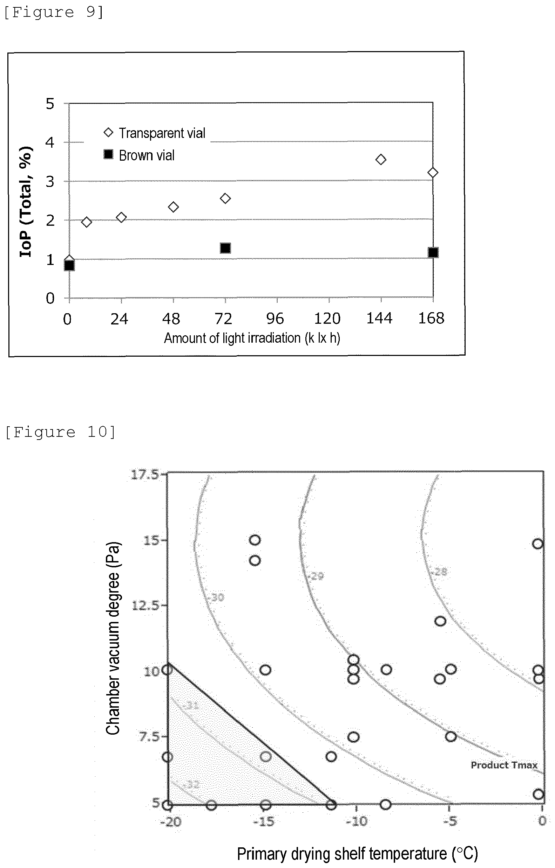

The results are shown in Table 9. It was found that the generation of aggregates (Figure 8) and IoP (Figure 9) can be more suppressed when brown glass vials were used compared with when transparent glass vials were used.

**[Table 9]**

| Test items | | Transparent vial | | | | | | | Brown vial | | |
|---|---|---|---|---|---|---|---|---|---|---|---|
| | | 1000 lx white fluorescent lamp | | | | | | | 1000 lx white fluorescent lamp | | |
| | | 0h | 8h | 24h | 48h | 72h | 144h | 168h | 0h | 72h | 168h |
| Protein content (mg/mL) | | 18.9 | 19.2 | 19.3 | 19.4 | 18.7 | 19.1 | 19.3 | 18.7 | 18.9 | 18.9 |
| pH | | 5.54 | 5.49 | 5.52 | 5.51 | 5.51 | 5.50 | 5.47 | 5.52 | 5.50 | 5.47 |
| DAR | | 7.6 | 7.6 | 7.6 | 7.6 | 7.6 | 7.6 | 7.6 | 7.8 | 7.6 | 7.7 |
| SEC (%) | Monomers | 98.5 | 96.5 | 95.2 | 94.0 | 93.1 | 91.7 | 91.6 | 98.8 | 97.3 | 96.8 |
| | Aggregates | 0.7 | 2.8 | 4.1 | 5.3 | 6.2 | 7.6 | 7.7 | 0.7 | 2.1 | 2.7 |
| | Fragments | 0.7 | 0.7 | 0.6 | 0.6 | 0.6 | 0.6 | 0.6 | 0.5 | 0.6 | 0.5 |
| NPI (%) | | 0.84 | 0.72 | 0.71 | 0.74 | 0.74 | 0.79 | 0.74 | 0.78 | 0.70 | 0.75 |
| IoP (%) | | 0.98 | 1.95 | 2.07 | 2.33 | 2.55 | 3.53 | 3.20 | 0.84 | 1.28 | 1.15 |

### [Example 10: Setting on Lyophilization Method]

Important physical properties as indices in setting a lyophilization method of injections are the freezing glass transition point (Tg') of the drug-containing aqueous solution to be lyophilized and the collapse temperature (Tc) of the obtained cake having a porous structure which is produced during lyophilization. By drying at a product temperature of equal to or less than Tg' or Tc, a lyophilized body having a cake-like porous structure is obtained. The drying process generally consists of a first drying process to sublimate free water in the frozen body and a secondary drying process to sublimate water bound to solutes (bound water). In the primary drying process, it is appropriate that the free water is sublimated at a product temperature that is equal to or less than Tg', and it is also possible to be sublimated at a product temperature that is equal to or less than Tc.

For an aqueous solution comprising antibody-drug conjugate (1) (20 mg/mL), 9% of sucrose, 25 mM of a histidine buffer, and 0.03% of polysorbate 80 and having a pH of 5.5 (hereinafter, referred to as "aqueous solution (1)"), Tg' is -31°C and Tc is -27°C.

The study on lyophilization methods was conducted by filling 5.2 to 5.5 mL of aqueous solution (1) into glass vials of about 10 mL volume.

### (1) Study on parameters of primary drying process

The most important parameters in the primary drying process are shelf temperature and chamber vacuum degree. Shape (stability) and redissolubility of the lyophilized cake and drying time are significantly affected by these parameters, thus shelf temperatures in the range of -20°C to 0°C and chamber vacuum degrees in the range of 5 Pa to 15Pa were studied by a designed experimental method. Lyophilization was carried out using the parameters shown in Table 9 (shelf temperature of the annealing was -7°C).

**[Table 10]**

| Process | Step No. | Shelf temperature (°C) | Time (min) | Chamber vacuum degree (Pa) |
|---|---|---|---|---|
| Freezing | 1 | 5 | 30 | Without vacuum |
| | 2 | 5 | 120 | |
| | 3 | -40 | 60 | |
| | 4 | -40 | 120 | |
| | 5 | -7 | 60 | |
| | 6 | -7 | 120 | |
| | 7 | -40 | 60 | |
| | 8 | -40 | 120 | |
| Primary drying | 9 | -20 to 0 | 60 | 5 to 15 |
| | 10 | -20 to 0 | 2400 to 4800 | |
| Secondary drying | 11 | 30 | 60 to 120 | Without vacuum control |
| | 12 | 30 | 480 to 600 | |
| Unloading | 13 | 5 | 60 | |

Figure 10 is a contour plot of the product temperature of aqueous solution (1) when varying the shelf temperature and the chamber vacuum degree in the primary drying process. The product temperature showed a general product temperature behavior in that, at conditions of low shelf temperature and high chamber vacuum degree, the product temperature during primary drying becomes lower, while at conditions of high shelf temperature and low chamber vacuum degree, the product temperature becomes higher. At shelf temperatures of - 20°C to 0°C and chamber vacuum degrees of 5 Pa to 15 Pa, the product temperature showed Tc or less. However, it was revealed that partial collapse occurs in the porous structure of the lyophilized cake at parameters other than the portion surrounded by a triangle, and the appearance of the lyophilized cake becomes contracted (shrinkage).

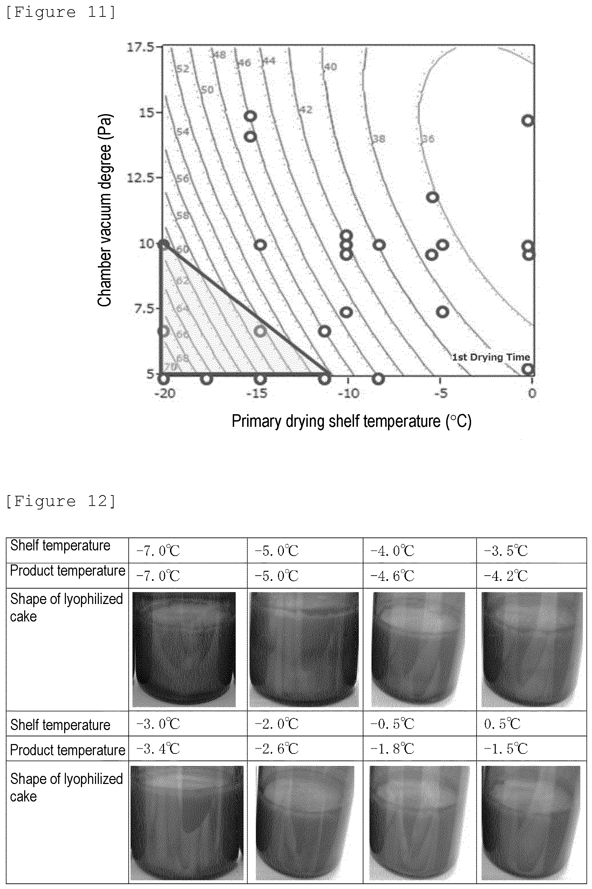

Figure 11 is a contour plot of drying times of aqueous solution (1) when varying the shelf temperature and the chamber vacuum degree in the primary drying process. The drying time showed a general drying rate behavior in that, at conditions of low shelf temperature and high chamber vacuum degree, the primary drying time becomes longer, while at conditions of high shelf temperature and low chamber vacuum degree, the drying time becomes shorter. The correlation between drying time and product temperature was observed. Regarding the parameter area of the portion surrounded by a triangle in the product temperature contour plot, it was revealed that it is possible to obtain a lyophilized cake of good shape, but a long primary drying process is required.

Since it is desired that the shape of the lyophilized cake has no shrinkage state in terms of appearance quality, it became obvious based on the result of this study that the primary drying process takes at least 58 hours. Thus, it was concluded that the total lyophilization time becomes 81 hours, since the freezing process requires at least 11 hours, and the secondary drying and unloading processes require at least 12 hours. In other words, it was estimated that the process period of lyophilization requires 3 days or more, and that the manufacturing time requires 5 days by adding the start date and end date for lyophilization.

However, a short time or short period is desired for lyophilization in terms of production efficiency and production cost. Thus, the necessity of the long-time lyophilization process was considered as a problem.

To solve this problem, and to obtain a lyophilized cake having less shrinkage even when the primary drying process time is shortened, a study on annealing parameters was conducted with focus on the annealing step which can be expected to improve the porous structure of the lyophilized cake.

### (2) Parameter settings on annealing

An important parameter in the annealing process is shelf temperature. By varying the shelf temperature, it is possible to adjust the product temperature of the frozen body.

In this study, optimization of the shelf temperature in annealing was conducted considering the porous structure of the lyophilized cake which is made by sublimation of ice in ice crystals. The shelf temperature of the annealing had been conventionally set so that there is a safety margin from the eutectic point. Considering this, the annealing was performed at a shelf temperature of -7°C since the eutectic point of aqueous solution (1) is -2.5°C. In addition, for growing ice crystals in the frozen body and reducing the sublimation resistance of the ice to proceed drying smoothly, and then obtaining a porous structure having a small specific surface area that hardly causes shrinkage in the lyophilized cake, annealing at shelf temperatures of higher than -7°C were also attempted. Accordingly, with setting the range of shelf temperatures in annealing from -7°C to 0.5°C to make a porous structure having small specific surface area, improvements in the appearance quality of the lyophilized cake were studied. The lyophilization of this study was performed by employing the parameters in Table 11, and the primary drying time was set to a total of about 42 hours.

**[Table 11]**

| Process | Step No. | Shelf temperature (°C) | Time (min) | Chamber vacuum degree (Pa) |
|---|---|---|---|---|
| Freezing | 1 | 5 | 30 | Without vacuum |
| | 2 | 5 | 120 | |
| | 3 | -40 | 60 | |
| | 4 | -40 | 120 | |
| | 5 | -7.0 to 0.5 | 60 | |
| | 6 | -7.0 to 0.5 | 120 | |
| | 7 | -40 | 60 | |
| | 8 | -40 | 120 | |
| Primary drying | 9 | -15 | 60 | 5 |
| | 10 | -15 | 300 | |
| | 11 | -10 | 30 | |
| | 12 | -10 | 300 | |
| | 13 | -5 | 30 | |
| | 14 | -5 | 300 | |
| | 15 | 0 | 30 | |
| | 16 | 0 | 1500 | |
| Secondary drying | 17 | 40 | 60 | 5 |
| | 18 | 40 | 600 | |
| Unloading | 19 | 5 | 60 | 5 |

The annealing was performed at shelf temperatures in the range of -7°C to 0.5°C. The product temperature (at annealing) and the shape of the lyophilized cake, when lyophilization was made, were shown in Figure 12. When the product temperature at annealing was in the range of -7.0°C to -4.6°C, remarkable shrinkage was observed in the lyophilized cake from the vial bottom towards the lower side, and when the product temperature was in the range of -4.2°C to -3.4°C, slight shrinkage was observed. However, when the product temperature was in the range of -2.6°C to -1.5°C, a lyophilized cake having no shrinkage was obtained. From these results, it was revealed that as the annealing is performed at a shelf temperature which is closer to the eutectic point, the shrinkage of the lyophilized cake decreases, and when the annealing is performed at a shelf temperature which is near the eutectic point, a lyophilized cake having no shrinkage can be obtained. Furthermore, it was revealed that, even when the product temperature is raised to -1.5°C that is above the eutectic point, the solid state is maintained and a lyophilized cake in normal shape can be obtained.

While the annealing had been conventionally performed at a shelf temperature about 5°C below the eutectic point, it has been found from the present study that, by annealing at a temperature near the eutectic point, a lyophilized cake having a better appearance can be obtained. From the result of this study, the shelf temperature of the annealing was set to -4°C to -2°C which are temperatures near the eutectic point.

Furthermore, from this study, it has been found that, annealing at a temperature near the eutectic point, the time of primary drying process can be shortened by about 40 hours. Based on the results of Figures 10 and 11, a shelf temperature of -5°C and a chamber vacuum degree of 7 Pa was set as the primary drying parameters because it was considered as a condition that allows drying to be conducted with a relatively low product temperature and is expected to shorten the drying time by about 18 hours.

### (3) Parameter settings on secondary drying process

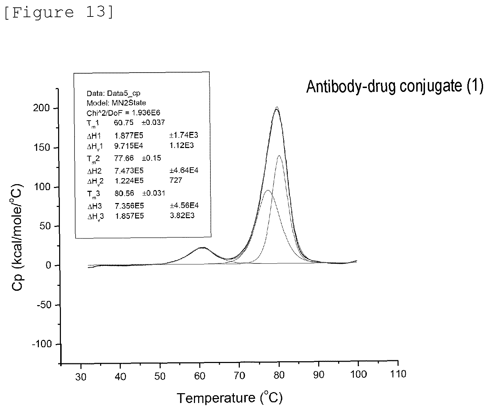

The secondary drying process is a process for drying the water bound to the solute (bound water), and it is generally known that the drying degree after secondary drying greatly affects the storage stability of a lyophilized injection. While bound water can be decreased as the drying is conducted at higher temperatures, the antibody part of an antibody-drug conjugate is generally labile to heat. In view of this, thermal denaturation onset of proteins was measured by a capillary differential scanning calorimeter. As shown in Figure 13, the thermal denaturation onset of antibody-drug conjugate (1) was 52°C to 53°C, thus the dryness (residual moisture) was evaluated while setting the shelf temperature of the secondary drying to 50°C or less.

With shelf temperatures of the secondary drying in the range of 30°C to 50°C, evaluation of dryness was performed. The lyophilization of this study was performed by employing the parameters of Table 12.

**[Table 12]**

| Process | Step No. | Shelf temperature (°C) | Time (min) | Chamber vacuum degree (Pa) |
|---|---|---|---|---|
| Freezing | 1 | 5 | 30 | Without vacuum |
| | 2 | 5 | 120 | |
| | 3 | -40 | 60 | |
| | 4 | -40 | 120 | |
| | 5 | -7 | 60 | |
| | 6 | -7 | 120 | |
| | 7 | -40 | 60 | |
| | 8 | -40 | 120 | |
| Primary drying | 9 | -10 to 0 | 60 | 7.5 to 15 |
| | 10 | -10 to 0 | 2400 | |
| Secondary drying | 11 | 30 to 50 | 60 | Without vacuum control |
| | 12 | 30 to 50 | 600 | |
| Unloading | 13 | 5 | 60 | |

The results of the residual moisture in lyophilized injections at each shelf temperature of the secondary drying are shown in Table 13. It had been confirmed that stability is maintained until the residual moisture in the lyophilized injection is 5%. However, for targeting a higher level of stability, the residual moisture in the formulation was set to 1% or less. Although the residual moisture is more or less affected by primary drying conditions, it was confirmed that the content of the residual moisture becomes 1% or less when the shelf temperature of the secondary drying is in the range of 40°C to 50°C. Based on the thermal denaturation onset and the results of this study, an intermediate value of 45°C in the range of 40°C to 50°C was set as the shelf temperature of the secondary drying.

**[Table 13]**

| Secondary drying shelf temperature (°C) | Residual moisture (%) | Primary drying | |
|---|---|---|---|
| | | Shelf temperature (°C) | Chamber vacuum degree (Pa) |
| 30 | 1.1 | -10 | 7.5 |
| | 1.2 | 0 | 7.5 |
| | 1.7 | -10 | 15 |
| | 1.9 | 0 | 15 |
| 40 | 0.8 | -10 | 7.5 |
| | 1.1 | -10 | 15 |
| | 0.9 | -5 | 10 |
| | 1.0 | 0 | 7.5 |
| | 1.2 | 0 | 15 |
| 50 | 0.4 | -10 | 7.5 |
| | 0.7 | 0 | 7.5 |
| | 0.6 | -10 | 15 |
| | 0.9 | 0 | 15 |

### (4) Summary

Excipients in lyophilized injections of biopharmaceuticals (protein pharmaceuticals) have a cryoprotective action on proteins, and non-reducing disaccharides are generally selected in pharmaceutical formulations, such as sucrose and trehalose which have no risk of Maillard reaction during storage. These disaccharides have lower Tg' and Tc than other saccharides, thus they are required to be dried at low shelf temperatures so that the product temperature does not exceed Tg' and Tc. Thus, there are problems that the lyophilization process becomes longer, and that the lyophilized cake tends to have shrink. The aqueous solution (1) was also considered to have the same problems, thus, for solving these problems, studies on the first drying, the annealing process and the first drying process were conducted, and based on the results of these studies, the lyophilization method of aqueous solution (1) was set up as shown in Table 14. A feature of this lyophilization method is the annealing parameters, and it has been found that, by annealing at a temperature near the eutectic point, a lyophilized cake having significantly reduced shrinkage can be produced in a short time.

**[Table 14]**

| Process | Step No. | Shelf temperature (°C) | Time (min) | Chamber vacuum degree (Pa) |
|---|---|---|---|---|
| Freezing | 1 | 5 | 30 | Without vacuum |
| | 2 | 5 | 120 | |
| | 3 | -40 | 60 | |
| | 4 | -40 | 120 | |
| | 5 | -4 to -2 | 60 | |
| | 6 | -4 to -2 | 120 | |
| | 7 | -40 | 60 | |
| | 8 | -40 | 120 | |
| Primary drying | 9 | -5 | 60 | 7 |
| | 10 | -5 | 2400 | |
| Secondary drying | 11 | 45 | 180 | 7 |
| | 12 | 45 | 480 | |
| Unloading | 13 | 5 | 60 | |

For lyophilized injections produced by the present lyophilization method, storage stabilities at 1 month under 50°C, 3 months under 40°C/75% RH, 3 months under 25°C/60% RH, and 3 months under 5°C were evaluated by employing protein concentration, moisture, insoluble microparticles, DAR, ratios of monomers, aggregates and fragments, NPI, IoP, charge isomers, and pH as indices. As a result, it was revealed that no significant quality changes are observed.

### [Example 11: Evaluation of long-term storage stability]

For the lyophilized injection produced in Example 10, storage stabilities at 6 months under 40°C/75% RH, 12 months under 25°C/60% RH, and 18 months under 5°C were evaluated by employing protein concentration, moisture, insoluble microparticles, DAR, ratios of monomers, aggregates and fragments, NPI, IoP, charge isomers, and pH as indices. As a result, it was revealed that no significant quality changes are observed.

### [Example 12: Additional Study on Lyophilized Method]

### (1) Parameter setting on annealing

As the lyophilizing method of aqueous solutions (1), for growing ice crystals in the frozen body, a method for performing annealing while holding the shelf temperature at -2.5°C for 120 minutes was employed. However, it was revealed that 120 minutes could not raise the temperature of the upper portion of the frozen body to -2.5°C so that ice crystal growth becomes insufficient and the lyophilized cake slightly shrinks. Furthermore, it was revealed that when the annealing time is extended to 240 minutes, the temperature of the upper portion of the frozen body reaches -2.5°C, thus the setting of the annealing time was changed to 4 hours. It has been revealed that, by changing the annealing time to 4 hours, ice crystal growth becomes sufficient, so the sublimation rate of water becomes faster and the primary drying time is shortened.

### (2) Parameter setting on primary drying process

Annealing was performed at -2.5°C for 4 hours, and primary drying process parameters were studied for shelf temperatures from -10°C to 15°C and for chamber vacuum degrees from 4 Pa to 30 Pa. Lyophilization was carried out with the parameters of Table 15.

**[Table 15]**

| Process | Step No. | Shelf temperature (°C) | Time (min) | Chamber vacuum degree (Pa) |
|---|---|---|---|---|
| Freezing | 1 | 5 | 120 | Without vacuum |
| | 2 | -40 | 60 | |
| | 3 | -40 | 120 | |
| | 4 | -2.5 | 60 | |
| | 5 | -2.5 | 240 | |
| | 6 | -40 | 60 | |
| | 7 | -40 | 120 | |
| Primary drying | 8 | -10 to 15 | 60 | 4 to 30 |
| | 9 | -10 to 15 | 2100 3840 | |
| Secondary drying | 10 | 45 | 180 | 4 to 30 |
| | 11 | 45 | 480 | |
| Unloading | 12 | 5 | 60 | 4 to 30 |
| | 13 | 5 | 3600 | |

When the shelf temperature was in the range of -10°C to 10°C and the chamber vacuum degree was 4 Pa to 30 Pa, the product temperature was Tc or less, and the appearance of the lyophilized cake was a cake shape having no shrinkage or collapse.

In particular, a shelf temperature of -5°C to 5°C and a chamber vacuum degree of 5 Pa to 15 Pa were considered as particularly safe ranges, and also in view of the drying time, a shelf temperature of 0°C and a chamber vacuum degree of 10 Pa, which were the intermediate conditions of these ranges, were set as suitable conditions.

### (3) Summary

Based on the above results, the lyophilization method of aqueous solution (1) was set as shown in Table 16.

**[Table 16]**

| Process | Step No. | Shelf temperature (°C) | Time (min) | Chamber vacuum degree (Pa) |
|---|---|---|---|---|
| Freezing | 1 | 5 | 120 | Without vacuum |
| | 2 | -40 | 60 | |
| | 3 | -40 | 120 | |
| | 4 | -2.5 | 60 | |
| | 5 | -2.5 | 240 | |
| | 6 | -40 | 60 | |
| | 7 | -40 | 120 | |
| Primary drying | 8 | 0 | 60 | 10 |
| | 9 | 0 | 2400 | |
| Secondary drying | 10 | 45 | 180 | 10 |
| | 11 | 45 | 480 | |
| Unloading | 12 | 5 | 60 | 10 |

For lyophilized injection produced by the present lyophilization method, storage stability at 3 months under 40°C/75% RH was evaluated by employing protein concentration, moisture, insoluble microparticles, DAR, ratios of monomers, aggregates and fragments, NPI, IoP, charge isomers, and pH as indices. Significant changes in quality from the starting time have not been observed.

### [Example 13: Study of other formulations and lyophilization conditions]

### (1) Preparation of the formulation

Active pharmaceutical ingredients (antibody-drug conjugate (1) (20 mg/mL), 25 mM of histidine, 9% of sucrose, pH 5.5) were dispensed into a UF membrane kit (AMICON ULTRA-15, 30 kDa) and centrifuged at a setting of 3000 rpm and 5°C to concentrate to about 3 times. The obtained concentrated active pharmaceutical ingredients were injected into a dialysis cassette (dialysis membrane, Slide-A-lyzer, MWCO 20,000), and dialyzed with a buffer of purpose formulation to 50 times or more volume of the sample injected into the dialysis cassette. The dialysis was performed twice for more than 5 hours at 5°C. After the dialysis, concentration or dilution was appropriately carried out so that the protein concentration became about 5 mg/mL or about 50 mg/mL, and then polysorbate 80 was added and mixed to prepare an aqueous solution comprising antibody-drug conjugate (1) (5 mg/mL), 8% of sucrose, 20 mM of a histidine buffer, and 0.04% of polysorbate 80, and having a pH of 4.0 (hereinafter, referred to as "aqueous solution (2)"); an aqueous solution comprising antibody-drug conjugate (1) (50 mg/mL), 6% of sucrose, 40 mM of the histidine buffer, and 0.04% of polysorbate 80, and having a pH of 7.0 (hereinafter, referred to as "aqueous solution (3)"); an aqueous solution comprising antibody-drug conjugate (1) (5 mg/mL), 8% of trehalose hydrate, 20 mM of a histidine buffer, and 0.04% of polysorbate 80, and having a pH of 4.0 (hereinafter, referred to as "aqueous solution (4)"); and an aqueous solution comprising antibody-drug conjugate (1) (50 mg/mL), 6% of trehalose hydrate, 40 mM of histidine buffer, and 0.04% of polysorbate 80, and having a pH of 7.0 (hereinafter, referred to as "aqueous solution (5)").

When expressed per 20 mg of antibody-drug conjugate (1), the aqueous solution (2) comprises 320 mg of sucrose, 80 mM of the histidine buffer, and 1.6 mg of polysorbate 80; the aqueous solution (3) comprises 24 mg of sucrose, 16 mM of the histidine buffer, and 0.16 mg of polysorbate 80; the aqueous solution (4) comprises 320 mg of trehalose hydrate, 80 mM of the histidine buffer, and 1.6 mg of polysorbate 80; and the aqueous solution (5) comprises 24 mg of trehalose hydrate, 16 mM of the histidine buffer, and 0.16 mg of polysorbate 80.

These solutions were filtered through a 0.22 µm filter. The filtered solutions each were filled into a brown glass vial by 5 mL, and in the case of the lyophilized formulation, the vials were half stoppered with rubber plugs, and lyophilization was carried out under the conditions shown in Table 17. After the lyophilization, the vials stoppered with the rubber plugs were sealed with caps.

**[Table 17]**

| Process | Step No. | Shelf temperature (°C) | Time (min) | Chamber vacuum degree (Pa) |
|---|---|---|---|---|
| Freezing | 1 | 5 | 30 | Without vacuum |
| | 2 | -40 | 60 | |
| | 3 | -40 | 120 | |
| | 4 | -1 | 60 | |
| | 5 | -1 | 240 | |
| | 6 | -40 | 60 | |
| | 7 | -40 | 120 | |
| Primary drying | 8 | -20 to 10 | 60 | 4 to 30 |
| | 9 | -20 to 10 | 1800 to 3600 | |
| Secondary drying | 10 | 45 | 180 | 4 to 30 |
| | 11 | 45 | 780 | |
| Unloading | 12 | 5 | 60 | 4 to 30 |
| | 13 | 5 | 3600 | |

### (2) Stability test

For lyophilized injections produced by the present lyophilization method, storage stability at 40°C/75% RH was evaluated by employing protein concentration, moisture, insoluble microparticles, DAR, ratios of monomers, aggregates and fragments, NPI, IoP, charge isomers, and pH as indices.

### [Example 14: Study on production and formulation of antibody-drug conjugate (2)]]

With reference to a production method described in International Publication No. WO 2015/155998, using an anti-HER3 antibody (an antibody comprising a heavy chain consisting of an amino acid sequence represented by SEQ ID NO: 3 and a light chain consisting an amino acid sequence represented by SEQ ID NO: 4), an antibody-drug conjugate in which a drug-linker represented by the following formula: 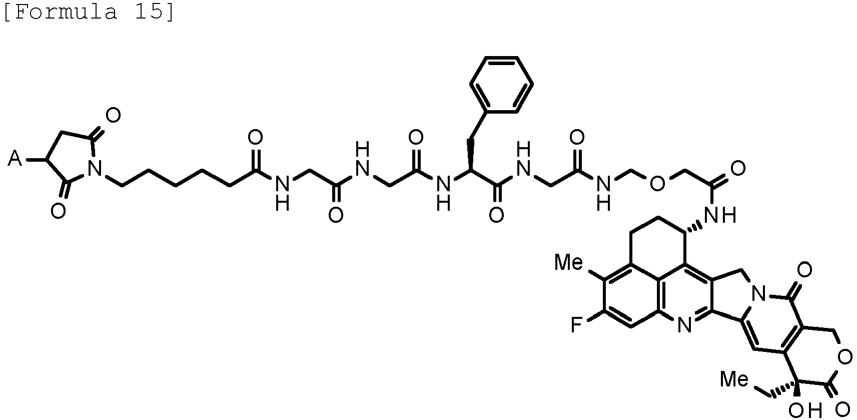 wherein A represents a connecting position to an antibody, is conjugated to the antibody via a thioether bond (hereinafter referred to as "antibody-drug conjugate (2)") was produced. The DAR of antibody-drug conjugate (2) was 7.7.

For antibody-drug conjugate (2), screening on formulations (20 mg/mL of antibody-drug conjugate (2), 9% of sucrose, 25 mM of the histidine buffer, 0.01 to 0.1% of polysorbate 20, pH 4.9 to 5.9) was performed by a method similar to the methods of Examples 2 to 8. As a result, it was found that an aqueous solution comprising antibody-drug conjugate (2) (20 mg/mL), 9% of sucrose, 25 mM of the histidine buffer, and 0.03% of polysorbate 20, and having a pH of 5.4 (hereinafter, referred to as "aqueous solution (6)") is a preferred formulation.

### [Example 15: Study on production and formulation of antibody-drug conjugate (3)]

With reference to a production method described in International Publication No. WO 2015/098099, using an anti-TROP2 antibody (an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6), an antibody-drug conjugate in which a drug-linker represented by the following formula: 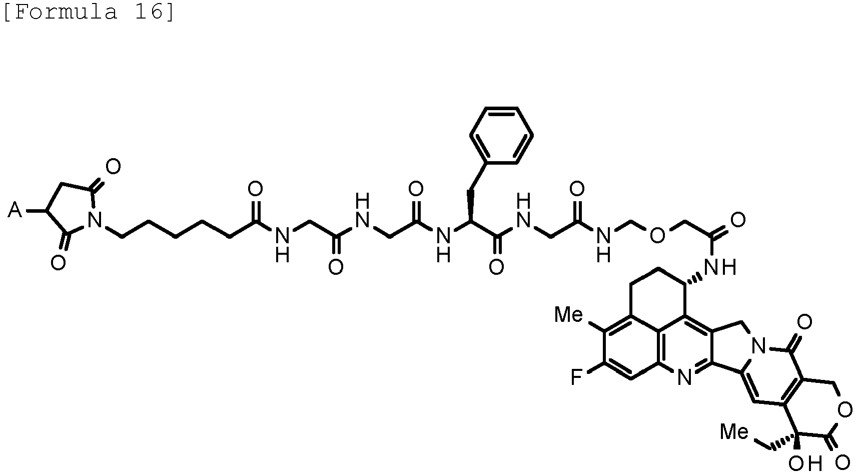 wherein A represents a connecting position to an antibody, is conjugated to the anti-TROP2 antibody via a thioether bond (hereinafter referred to as "antibody-drug conjugate (3)") was produced. The DAR of antibody-drug conjugate (3) was 4.3.

For antibody-drug conjugate (3), screening on formulations (antibody-drug conjugate (3) (20 mg/mL), sucrose (3 to 9%), histidine buffer (3 to 15 mM), polysorbate 20 or polysorbate 80 (0.01 to 0.1%), pH 5 to 7; or antibody-drug conjugate (3) (15 to 40 mg/mL), 9% of sucrose, 10 mM of the histidine buffer, 0.02% of polysorbate 80, pH 6.0) was performed in the method similar to the methods of Examples 2 to 8. As a result, it was found that an aqueous solution comprising antibody-drug conjugate (3) (20 mg/mL), 9% of sucrose, 10 mM of the histidine buffer, and 0.02% of polysorbate 80, and having a pH of 6.0 (hereinafter, referred to as "aqueous solution (7)"); as well as an aqueous solution comprising antibody-drug conjugate (3) (20 mg / mL), 9% of sucrose, 10 mM of the histidine buffer, and 0.03% of polysorbate 80, and having a pH of 6.0 (hereinafter, referred to as "aqueous solution (8)") are preferred formulations.

### [Example 16: Study on production and formulation of antibody-drug conjugate (4)]

With reference to a production method described in International Publication No. WO 2014/057687, using an anti-B7-H3 antibody (an antibody comprising a heavy chain consisting of an amino acid sequence consisting of amino acid residues 20 to 470 of SEQ ID NO: 7 and a light chain consisting of an amino acid sequence consisting of amino acid residues 21 to 233 of SEQ ID NO: 8), an antibody-drug conjugate in which a drug-linker represented by the following formula: 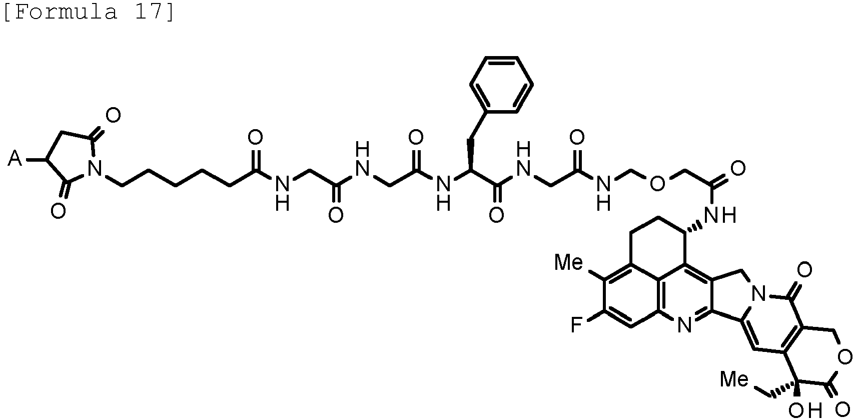 wherein A represents a connecting position to an antibody, is conjugated to the anti-B7-H3 antibody via a thioether bond (hereinafter referred to as "antibody-drug conjugate (4)") was produced. The DAR of antibody-drug conjugate (4) was 4.6.

For antibody-drug conjugate (4), screening on formulations (Antibody-drug conjugate (4) (20 mg/mL), 9% of sucrose or 10% of trehalose hydrate, 10 mM of a histidine buffer or 10 mM of a succinate buffer, polysorbate 20 or polysorbate 80 (0.005 to 0.04%), pH 5.2 to 6.2; or antibody-drug conjugate (4) (20 to 60 mg/mL), 9% of sucrose, 10 mM of the histidine buffer, polysorbate 20 (0.005 to 0.04%), pH 5.2 to 6.2) was performed in the method similar to the methods of Examples 2 to 8. As a result, it was found that an aqueous solution comprising antibody-drug conjugate (4) (20 mg/mL), 9% of sucrose, 10 mM of the histidine buffer, and 0.02% polysorbate 20, and having a pH of 5.9 (hereinafter, referred to as "aqueous solution (9)"); as well as an aqueous solution comprising antibody-drug conjugate (4) (20 mg/mL), 9% of sucrose, 10 mM histidine buffer, and 0.03% polysorbate 20, and having a pH of 5.9 (hereinafter, referred to as "aqueous solution (10)") are preferred formulations.

### [Production Example 17: Study on production and formulation of antibody-drug conjugate (5)]

With reference to a production method described in International Publication No. WO 2018/135501, using an anti-GPR20 antibody (an antibody comprising a heavy chain consisting of an amino acid sequence consisting of amino acid residues 20 to 470 of SEQ ID NO: 9 and a light chain consisting of an amino acid sequence consisting of amino acid residues 21 to 234 of SEQ ID NO: 10), an antibody-drug conjugate in which a drug-linker represented by the following formula: 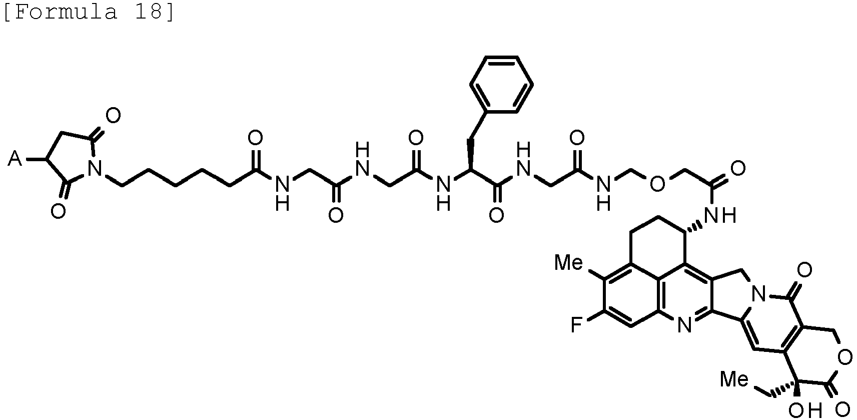 wherein A represents a connecting position to an antibody, is conjugated to the anti-GPR20 antibody via a thioether bond (hereinafter referred to as "antibody-drug conjugate (5)") was produced. The DAR of antibody-drug conjugate (5) was 7.8.

For antibody-drug conjugate (5), screening on a formulation (Antibody-drug conjugate (5) (20 to 60 mg/mL), 9% of sucrose, 10 mM of the histidine buffer, and polysorbate 80 (0.01 to 0.1%), pH 5.0 to 6.5) was performed in the method similar to the methods of Examples 2 to 8. As a result, it was found that an aqueous solution comprising antibody-drug conjugate (5) (20 mg/mL), 9% of sucrose, 10 mM of the histidine buffer, and 0.03% of polysorbate 80, and having a pH of 5.4 (hereinafter, referred to as "aqueous solution (11)") is a preferred formulation.

### [Example 18: Preparation of lyophilized injection and stability test]

The aqueous solutions (6) to (11) adjusted in Examples 14 to 17 were lyophilized in a similar manner to that described in Examples 10 and 12, respectively to produce lyophilized injections.

For these obtained lyophilized injections, the storage stability at 1 month and 3 months under 40°C/75% RH was evaluated by employing protein concentration, moisture, insoluble microparticles, DAR, ratios of monomers, aggregates and fragments, NPI, IoP, charge isomers, and pH as indices. Significant changes in quality from the starting time have not been observed.

### Free Text of Sequence Listing

SEQ ID NO: 1 - Amino acid sequence of a heavy chain of the anti-HER2 antibody
SEQ ID NO: 2 - Amino acid sequence of a light chain of the anti-HER2 antibody
SEQ ID NO: 3 - Amino acid sequence of a heavy chain of the anti-HER3 antibody
SEQ ID NO: 4 - Amino acid sequence of a light chain of the anti-HER3 antibody
SEQ ID NO: 5 - Amino acid sequence of a heavy chain of the anti-TROP2 antibody
SEQ ID NO: 6 - Amino acid sequence of a light chain of the anti-TROP2 antibody
SEQ ID NO: 7 - Amino acid sequence of a heavy chain of the anti-B7-H3 antibody
SEQ ID NO: 8 - Amino acid sequence of a light chain of the anti-B7-H3 antibody
SEQ ID NO: 9 - Amino acid sequence of a heavy chain of the anti-GPR20 antibody
SEQ ID NO: 10 - Amino acid sequence of a light chain of the anti-GPR20 antibody

## Claims

1. A pharmaceutical composition comprising
(i) an antibody-drug conjugate,
(ii) a histidine buffer,
(iii) sucrose or trehalose, and
(iv) a surfactant,
wherein the antibody-drug conjugate is an antibody-drug conjugate in which a drug-linker represented by the following formula: 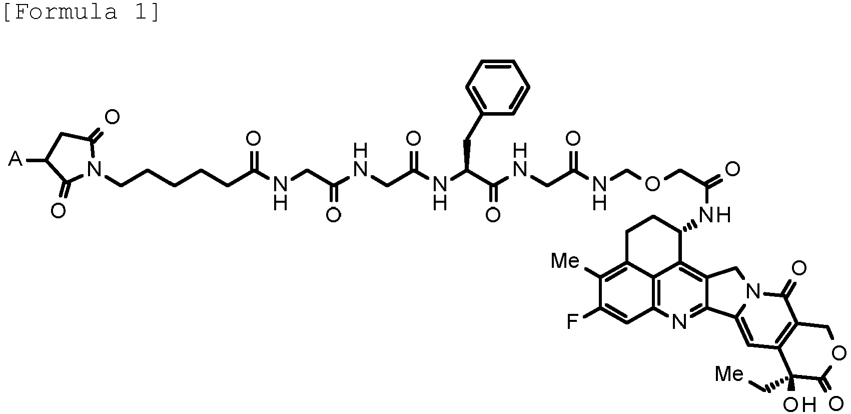 wherein A represents a connecting position to an antibody, is conjugated to the antibody via a thioether bond.

2. The pharmaceutical composition according to claim 1, wherein the surfactant is polysorbate 80 or polysorbate 20.

3. The pharmaceutical composition according to claim 1 or 2 comprising,
(i) per 20 mg of the antibody-drug conjugate,
(ii) 3 to 80 mmol of the histidine buffer,
(iii) 24 to 320 mg of sucrose or trehalose, and
(iv) 0.05 to 1.6 mg of polysorbate 80 or polysorbate 20.

4. The pharmaceutical composition according to any one of claims 1 to 3 comprising,
(i) per 20 mg of the antibody-drug conjugate,
(ii) 10 to 40 mmol of the histidine buffer,
(iii) 90 mg of sucrose or 100 mg of trehalose hydrate, and
(iv) 0.2 to 0.4 mg of polysorbate 80 or polysorbate 20.

5. The pharmaceutical composition according to any one of claims 1 to 4 comprising,
(i) per 20 mg of the antibody-drug conjugate,
(ii) 10 or 25 mmol of the histidine buffer,
(iii) 90 mg of sucrose, and
(iv) 0.2 or 0.3 mg of polysorbate 80 or polysorbate 20.

6. The pharmaceutical composition according to any one of claims 1 to 5, wherein the pH of the composition when the antibody-drug conjugate is dissolved in water at a concentration of 20 mg/mL is 4.0 to 7.0.

7. The pharmaceutical composition according to any one of claims 1 to 6, wherein the antibody in the antibody-drug conjugate is an anti-HER2 antibody, an anti-HER3 antibody, an anti-TROP2 antibody, an anti-B7-H3 antibody, or an anti-GPR20 antibody.

8. The pharmaceutical composition according to claim 7, wherein the antibody in the antibody-drug conjugate is an anti-HER2 antibody.

9. The pharmaceutical composition according to claim 8, wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2, or an antibody comprising a heavy chain consisting of an amino acid sequence represented by SEQ ID NO: 1 and a light chain consisting of an amino acid sequence represented by SEQ ID NO: 2.

10. The pharmaceutical composition according to claim 8 or 9, wherein the average number of units of the drug-linker conjugated per antibody molecule in the antibody-drug conjugate is in the range of from 7 to 8.

11. The pharmaceutical composition according to any one of claims 8 to 10 comprising,
(i) per 20 mg of the antibody-drug conjugate,
(ii) 25 mmol of the histidine buffer,
(iii) 90 mg of sucrose, and
(iv) 0.3 mg of polysorbate 80,
wherein the pH of the composition when the antibody-drug conjugate is dissolved in water at a concentration of 20 mg/mL is 5.5.

12. The pharmaceutical composition according to any one of claims 8 to 10 comprising,
(i) per 20 mg of the antibody-drug conjugate,
(ii) 0.89 mg of L-histidine and 4.04 mg of L-histidine hydrochloride hydrate,
(iii) 90 mg of sucrose, and
(iv) 0.3 mg of polysorbate 80.

13. The pharmaceutical composition according to any one of claims 8 to 10 comprising
(i) 100 mg of the antibody-drug conjugate,
(ii) 4.45 mg of L-histidine and 20.2 mg of L-histidine hydrochloride hydrate,
(iii) 450 mg of sucrose, and
(iv) 1.5 mg of polysorbate 80.

14. The pharmaceutical composition according to claim 7, wherein the antibody in the antibody-drug conjugate is an anti-HER3 antibody.

15. The pharmaceutical composition according to claim 14, wherein the anti-HER3 antibody is an antibody comprising a heavy chain consisting of an amino acid sequence represented by SEQ ID NO: 3 and a light chain consisting of an amino acid sequence represented by SEQ ID NO: 4, or a variant of the antibody in which a lysine residue at the carboxyl terminus of the heavy chain is deleted.

16. The pharmaceutical composition according to claim 14 or 15, wherein the average number of units of the drug-linker conjugated per antibody molecule in the antibody-drug conjugate is in the range of from 7 to 8.

17. The pharmaceutical composition according to any one of claims 14 to 16 comprising,
(i) per 20 mg of the antibody-drug conjugate,
(ii) 25 mmol of the histidine buffer,
(iii) 90 mg of sucrose, and
(iv) 0.3 mg of polysorbate 20,
wherein the pH of the composition when the antibody-drug conjugate is dissolved in water at a concentration of 20 mg/mL is 5.4.

18. The pharmaceutical composition according to any one of claims 14 to 16 comprising,
(i) per 20 mg of the antibody-drug conjugate,
(ii) 0.81 mg of L-histidine and 4.14 mg of L-histidine hydrochloride hydrate,
(iii) 90 mg of sucrose, and
(iv) 0.3 mg of polysorbate 20.

19. The pharmaceutical composition according to any one of claims 14 to 16 comprising
(i) 100 mg of the antibody-drug conjugate,
(ii) 4.06 mg of L-histidine and 20.7 mg of L-histidine hydrochloride hydrate,
(iii) 450 mg of sucrose, and
(iv) 1.5 mg of polysorbate 20.

20. The pharmaceutical composition according to claim 7, wherein the antibody in the antibody-drug conjugate is an anti-TROP2 antibody.

21. The pharmaceutical composition according to claim 20, wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6, or a variant of the antibody in which a lysine residue at the carboxyl terminus of the heavy chain is deleted.

22. The pharmaceutical composition according to claim 20 or 21, wherein the average number of units of the drug-linker conjugated per antibody molecule in the antibody-drug conjugate is in the range of from 3 to 5.

23. The pharmaceutical composition according to any one of claims 20 to 22 comprising,
(i) per 20 mg of the antibody-drug conjugate,
(ii) 10 mmol of the histidine buffer,
(iii) 90 mg of sucrose, and
(iv) 0.2 or 0.3 mg of polysorbate 80,
wherein the pH of the composition when the antibody-drug conjugate is dissolved in water at a concentration of 20 mg/mL is 6.0.

24. The pharmaceutical composition according to any one of claims 20 to 22 comprising,
(i) per 20 mg of the antibody-drug conjugate,
(ii) 0.78 mg of L-histidine and 1.05 mg of L-histidine hydrochloride hydrate,
(iii) 90 mg of sucrose, and
(iv) 0.2 or 0.3 mg of polysorbate 80.

25. The pharmaceutical composition according to any one of claims 20 to 22 comprising
(i) 100 mg of the antibody-drug conjugate,
(ii) 3.88 mg of L-histidine and 5.26 mg of L-histidine hydrochloride hydrate,
(iii) 450 mg of sucrose, and
(iv) 1.0 or 1.5 mg of polysorbate 80.

26. The pharmaceutical composition according to claim 7, wherein the antibody in the antibody-drug conjugate is an anti-B7-H3 antibody.

27. The pharmaceutical composition according to claim 26, wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which a lysine residue at the carboxyl terminus of the heavy chain is deleted.

28. The pharmaceutical composition according to claim 26 or 27, wherein the average number of units of the drug-linker conjugated per antibody molecule in the antibody-drug conjugate is in the range of from 3 to 5.

29. The pharmaceutical composition according to any one of claims 26 to 28 comprising,
(i) per 20 mg of the antibody-drug conjugate,
(ii) 10 mmol of the histidine buffer,
(iii) 90 mg of sucrose, and
(iv) 0.2 or 0.3 mg of polysorbate 20,
wherein the pH of the composition when the antibody-drug conjugate is dissolved in water at a concentration of 20 mg/mL is 5.9.

30. The pharmaceutical composition according to any one of claims 26 to 28 comprising,
(i) per 20 mg of the antibody-drug conjugate,
(ii) 0.65 mg of L-histidine and 1.22 mg of L-histidine hydrochloride hydrate,
(iii) 90 mg of sucrose, and
(iv) 0.2 or 0.3 mg of polysorbate 20.

31. The pharmaceutical composition according to any one of claims 26 to 28 comprising
(i) 100 mg of the antibody-drug conjugate,
(ii) 3.23 mg of L-histidine and 6.12 mg of L-histidine hydrochloride hydrate,
(iii) 450 mg of sucrose, and
(iv) 1.0 or 1.5 mg of polysorbate 20.

32. The pharmaceutical composition according to claim 7, wherein the antibody in the antibody-drug conjugate is an anti-GPR20 antibody.

33. The pharmaceutical composition according to claim 32, wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10, or a variant of the antibody in which a lysine residue at the carboxyl terminus of the heavy chain is deleted.

34. The pharmaceutical composition according to claim 32 or 33, wherein the average number of units of the drug-linker conjugated per antibody molecule in the antibody-drug conjugate is in the range of from 7 to 8.

35. The pharmaceutical composition according to any one of claims 32 to 34 comprising,
(i) per 20 mg of the antibody-drug conjugate,
(ii) 10 mmol of the histidine buffer,
(iii) 90 mg of sucrose, and
(iv) 0.3 mg of polysorbate 80,
wherein the pH of the composition when the antibody-drug conjugate is dissolved in water at a concentration of 20 mg/mL is 5.4.

36. The pharmaceutical composition according to any one of claims 32 to 34 comprising,
(i) per 20 mg of the antibody-drug conjugate,
(ii) 0.32 mg of L-histidine and 1.66 mg of L-histidine hydrochloride hydrate,
(iii) 90 mg of sucrose, and
(iv) 0.3 mg of polysorbate 80.

37. The pharmaceutical composition according to any one of claims 32 to 34 comprising
(i) 100 mg of the antibody-drug conjugate,
(ii) 1.62 mg of L-histidine and 8.29 mg of L-histidine hydrochloride hydrate,
(iii) 450 mg of sucrose, and
(iv) 1.5 mg of polysorbate 80.

38. The pharmaceutical composition according to any one of claims 1 to 37, wherein the composition is in the form of an injection.

39. The pharmaceutical composition according to claim 38, wherein the composition is in the form of an aqueous injection.

40. The pharmaceutical composition according to claim 39, wherein the concentration of the antibody-drug conjugate is 20 mg/mL.

41. The pharmaceutical composition according to claim 39 or 40, wherein the composition is frozen.

42. The pharmaceutical composition according to claim 38, wherein the composition is in the form of a lyophilized injection.

43. The pharmaceutical composition according to claim 42, wherein the composition is stored in a brown vial.

44. The pharmaceutical composition according to any one of claims 1 to 43, wherein the composition is for treating cancer.

45. A method for producing a pharmaceutical composition according to claim 42, comprising the steps of:
(1) preparing an aqueous solution comprising predetermined amounts of
(i) an antibody-drug conjugate,
(ii) a histidine buffer,
(iii) sucrose or trehalose, and
(iv) a surfactant,
(2) if necessary, adjusting the pH of the aqueous solution to a predetermined value, and then
(3) lyophilizing the aqueous solution.

46. The production method according to claim 45, wherein the step of lyophilizing the aqueous solution comprises a process of annealing at a shelf temperature which is near the eutectic point of the aqueous solution,
wherein near the eutectic point indicates the range of from a temperature which is 1.5°C lower than the eutectic point to a temperature which is 1.5°C higher than the eutectic point.

47. The production method according to claim 46, **characterized in that** the time for a process of primary drying is shortened compared with that when annealing at a shelf temperature which is 5°C lower than the eutectic point is performed.

48. The production method according to claim 46 or 47, **characterized in that** a lyophilized cake having less shrinkage is obtained compared with that when annealing at a shelf temperature which is 5°C lower than the eutectic point is performed.

49. The production method according to claim 45, wherein the step of lyophilizing the aqueous solution comprises a process of performing annealing at a shelf temperature of -4 to -1°C.

50. The production method according to claim 49, wherein the step of lyophilizing the aqueous solution further comprises a process of primary drying at a shelf temperature of -5 to 5°C under a vacuum of 5 to 15 Pa.

51. The production method according to claim 50, wherein the step of lyophilizing the aqueous solution further comprises a process of secondary drying at a shelf temperature of 40 to 50°C under a vacuum of 5 to 15 Pa.


SEQUENCE LISTING

<110>  DAIICHI SANKYO COMPANY, LIMITED

<120>  FORMULATION OF ANTIBODY-DRUG CONJUGATE AND LYOPHILIZATION METHOD
THEREOF

<130>  DSPCT-FP1822

<150>  JP2017-160071
<151>  2017-08-23

<160>  10

<170>  PatentIn version 3.5

<210>  1
<211>  450
<212>  PRT
<213>  Artificial Sequence

<220>
<223>  Heavy chain of anti-HER2 antibody

<400>  1

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser

145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  2
<211>  214
<212>  PRT
<213>  Artificial Sequence

<220>
<223>  Light chain of anti-HER2 antibody

<400>  2

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln

145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  3
<211>  447
<212>  PRT
<213>  Artificial Sequence

<220>
<223>  Heavy chain of anti-HER3 antibody

<400>  3

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asn His Ser Gly Ser Thr Asn Tyr Asn Pro Ser Leu Lys
    50                  55                  60

Ser Arg Val Thr Ile Ser Val Glu Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Asp Lys Trp Thr Trp Tyr Phe Asp Leu Trp Gly Arg Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Arg Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser

385                 390                 395                 400

Asp Gly Ser Phe Phe Leu Tyr Ser Lys Leu Thr Val Asp Lys Ser Arg
            405                 410                 415

Trp Gln Gln Gly Asn Val Phe Ser Cys Ser Val Met His Glu Ala Leu
        420                 425                 430

His Asn His Tyr Thr Gln Lys Ser Leu Ser Leu Ser Pro Gly Lys
    435                 440                 445

<210>  4
<211>  220
<212>  PRT
<213>  Artificial Sequence

<220>
<223>  Light chain of anti-HER3 antibody

<400>  4

Asp Ile Glu Met Thr Gln Ser Pro Asp Ser Leu Ala Val Ser Leu Gly
1               5                   10                  15

Glu Arg Ala Thr Ile Asn Cys Arg Ser Ser Gln Ser Val Leu Tyr Ser
            20                  25                  30

Ser Ser Asn Arg Asn Tyr Leu Ala Trp Tyr Gln Gln Asn Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Thr Pro Arg Thr Phe Gly Gln Gly Thr Lys Val Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

<210>  5
<211>  470
<212>  PRT
<213>  Artificial Sequence

<220>
<223>  Heavy chain of anti-TROP2 antibody

<400>  5

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Thr Ala Gly Met Gln Trp Val Arg Gln Ala Pro Gly Gln Gly Leu
    50                  55                  60

Glu Trp Met Gly Trp Ile Asn Thr His Ser Gly Val Pro Lys Tyr Ala
65                  70                  75                  80

Glu Asp Phe Lys Gly Arg Val Thr Ile Ser Ala Asp Thr Ser Thr Ser
                85                  90                  95

Thr Ala Tyr Leu Gln Leu Ser Ser Leu Lys Ser Glu Asp Thr Ala Val
            100                 105                 110

Tyr Tyr Cys Ala Arg Ser Gly Phe Gly Ser Ser Tyr Trp Tyr Phe Asp
        115                 120                 125

Val Trp Gly Gln Gly Thr Leu Val Thr Val Ser Ser Ala Ser Thr Lys
    130                 135                 140

Gly Pro Ser Val Phe Pro Leu Ala Pro Ser Ser Lys Ser Thr Ser Gly
145                 150                 155                 160

Gly Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Arg Val Glu Pro
225                 230                 235                 240

Lys Ser Cys Asp Lys Thr His Thr Cys Pro Pro Cys Pro Ala Pro Glu
                245                 250                 255

Leu Leu Gly Gly Pro Ser Val Phe Leu Phe Pro Pro Lys Pro Lys Asp
            260                 265                 270

Thr Leu Met Ile Ser Arg Thr Pro Glu Val Thr Cys Val Val Val Asp
        275                 280                 285

Val Ser His Glu Asp Pro Glu Val Lys Phe Asn Trp Tyr Val Asp Gly
    290                 295                 300

Val Glu Val His Asn Ala Lys Thr Lys Pro Arg Glu Glu Gln Tyr Asn
305                 310                 315                 320

Ser Thr Tyr Arg Val Val Ser Val Leu Thr Val Leu His Gln Asp Trp
                325                 330                 335

Leu Asn Gly Lys Glu Tyr Lys Cys Lys Val Ser Asn Lys Ala Leu Pro
            340                 345                 350

Ala Pro Ile Glu Lys Thr Ile Ser Lys Ala Lys Gly Gln Pro Arg Glu
        355                 360                 365

Pro Gln Val Tyr Thr Leu Pro Pro Ser Arg Glu Glu Met Thr Lys Asn
    370                 375                 380

Gln Val Ser Leu Thr Cys Leu Val Lys Gly Phe Tyr Pro Ser Asp Ile
385                 390                 395                 400

Ala Val Glu Trp Glu Ser Asn Gly Gln Pro Glu Asn Asn Tyr Lys Thr
                405                 410                 415

Thr Pro Pro Val Leu Asp Ser Asp Gly Ser Phe Phe Leu Tyr Ser Lys
            420                 425                 430

Leu Thr Val Asp Lys Ser Arg Trp Gln Gln Gly Asn Val Phe Ser Cys
        435                 440                 445

Ser Val Met His Glu Ala Leu His Asn His Tyr Thr Gln Lys Ser Leu
    450                 455                 460

Ser Leu Ser Pro Gly Lys
465                 470

<210>  6
<211>  234
<212>  PRT
<213>  Artificial Sequence

<220>
<223>  Light chain of anti-TROP2 antibody

<400>  6

Met Val Leu Gln Thr Gln Val Phe Ile Ser Leu Leu Leu Trp Ile Ser
1               5                   10                  15

Gly Ala Tyr Gly Asp Ile Gln Met Thr Gln Ser Pro Ser Ser Leu Ser
            20                  25                  30

Ala Ser Val Gly Asp Arg Val Thr Ile Thr Cys Lys Ala Ser Gln Asp
        35                  40                  45

Val Ser Thr Ala Val Ala Trp Tyr Gln Gln Lys Pro Gly Lys Ala Pro
    50                  55                  60

Lys Leu Leu Ile Tyr Ser Ala Ser Tyr Arg Tyr Thr Gly Val Pro Ser
65                  70                  75                  80

Arg Phe Ser Gly Ser Gly Ser Gly Thr Asp Phe Thr Leu Thr Ile Ser
                85                  90                  95

Ser Leu Gln Pro Glu Asp Phe Ala Val Tyr Tyr Cys Gln Gln His Tyr
            100                 105                 110

Ile Thr Pro Leu Thr Phe Gly Gln Gly Thr Lys Leu Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  7
<211>  471
<212>  PRT
<213>  Artificial Sequence

<220>
<223>  Heavy chain of anti-B7-H3 antibody

<400>  7

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ser Ser Val Lys Val Ser Cys Lys Ala Ser Gly Tyr Thr Phe
        35                  40                  45

Thr Asn Tyr Val Met His Trp Val Arg Gln Ala Pro Gly Gln Gly Leu
    50                  55                  60

Glu Trp Met Gly Tyr Ile Asn Pro Tyr Asn Asp Asp Val Lys Tyr Asn
65                  70                  75                  80

Glu Lys Phe Lys Gly Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
        100                 105                 110

Tyr Tyr Cys Ala Arg Trp Gly Tyr Tyr Gly Ser Pro Leu Tyr Tyr Phe
    115                 120                 125

Asp Tyr Trp Gly Gln Gly Thr Leu Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
        180                 185                 190

Thr Phe Pro Ala Val Leu Gln Ser Ser Gly Leu Tyr Ser Leu Ser Ser
    195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Arg Val Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
        260                 265                 270

Asp Thr Leu Met Ile Ser Arg Thr Pro Glu Val Thr Cys Val Val Val
    275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
        340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Glu Glu Met Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470

<210>  8
<211>  233
<212>  PRT
<213>  Artificial Sequence

<220>
<223>  Light chain of anti-B7-H3 antibody

<400>  8

Met Val Leu Gln Thr Gln Val Phe Ile Ser Leu Leu Leu Trp Ile Ser
1               5                   10                  15

Gly Ala Tyr Gly Glu Ile Val Leu Thr Gln Ser Pro Ala Thr Leu Ser
            20                  25                  30

Leu Ser Pro Gly Glu Arg Ala Thr Leu Ser Cys Arg Ala Ser Ser Arg
        35                  40                  45

Leu Ile Tyr Met His Trp Tyr Gln Gln Lys Pro Gly Gln Ala Pro Arg
    50                  55                  60

Pro Leu Ile Tyr Ala Thr Ser Asn Leu Ala Ser Gly Ile Pro Ala Arg
65                  70                  75                  80

Phe Ser Gly Ser Gly Ser Gly Thr Asp Phe Thr Leu Thr Ile Ser Ser
                85                  90                  95

Leu Glu Pro Glu Asp Phe Ala Val Tyr Tyr Cys Gln Gln Trp Asn Ser
            100                 105                 110

Asn Pro Pro Thr Phe Gly Gln Gly Thr Lys Val Glu Ile Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230

<210>  9
<211>  472
<212>  PRT
<213>  Artificial Sequence

<220>
<223>  Heavy chain of anti-GPR20 antibody

<400>  9

Met Lys His Leu Trp Phe Phe Leu Leu Leu Val Ala Ala Pro Arg Trp
1               5                   10                  15

Val Leu Ser Glu Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr Tyr Ile Ser Trp Ile Arg Gln Ala Pro Gly Gln Gly Leu

50                  55                  60

Lys Tyr Met Gly Phe Ile Asn Pro Gly Ser Gly His Thr Asn Tyr Asn
65                  70                  75                  80

Glu Lys Phe Lys Gly Arg Val Thr Ile Thr Ala Asp Lys Ser Ser Ser
                85                  90                  95

Thr Ala Thr Met Glu Leu Ser Ser Leu Arg Ser Glu Asp Thr Ala Val
            100                 105                 110

Tyr Tyr Cys Ala Arg Gly Ala Gly Gly Phe Leu Arg Ile Ile Thr Lys
        115                 120                 125

Phe Asp Tyr Trp Gly Gln Gly Thr Leu Val Thr Val Ser Ser Ala Ser
    130                 135                 140

Thr Lys Gly Pro Ser Val Phe Pro Leu Ala Pro Ser Ser Lys Ser Thr
145                 150                 155                 160

Ser Gly Gly Thr Ala Ala Leu Gly Cys Leu Val Lys Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Arg Val
225                 230                 235                 240

Glu Pro Lys Ser Cys Asp Lys Thr His Thr Cys Pro Pro Cys Pro Ala
                245                 250                 255

Pro Glu Leu Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305                 310                 315                 320

Tyr Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Ala
            340                 345                 350

Leu Pro Ala Pro Ile Glu Lys Thr Ile Ser Lys Ala Lys Gly Gln Pro
        355                 360                 365

Arg Glu Pro Gln Val Tyr Thr Leu Pro Pro Ser Arg Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Lys Leu Thr Val Asp Lys Ser Arg Trp Gln Gln Gly Asn Val Phe
        435                 440                 445

Ser Cys Ser Val Met His Glu Ala Leu His Asn His Tyr Thr Gln Lys
    450                 455                 460

Ser Leu Ser Leu Ser Pro Gly Lys
465                 470

<210>  10
<211>  234
<212>  PRT
<213>  Artificial Sequence

<220>
<223>  Light chain of anti-GPR20 antibody

<400>  10

Met Val Leu Gln Thr Gln Val Phe Ile Ser Leu Leu Leu Trp Ile Ser
1               5                   10                  15

Gly Ala Tyr Gly Asp Thr Gln Leu Thr Gln Ser Pro Ser Ser Leu Ser
            20                  25                  30

Ala Ser Val Gly Asp Arg Val Thr Ile Thr Cys Arg Ala Ser Lys Ser

35                  40                  45

Val Ser Thr Tyr Ile His Trp Tyr Gln Gln Lys Pro Gly Lys Gln Pro
    50                  55                  60

Lys Leu Leu Ile Tyr Ser Ala Gly Asn Leu Glu Ser Gly Val Pro Ser
65                  70                  75                  80

Arg Phe Ser Gly Ser Gly Ser Gly Thr Asp Phe Thr Leu Thr Ile Ser
                85                  90                  95

Ser Leu Gln Pro Glu Asp Phe Ala Asn Tyr Tyr Cys Gln Gln Ile Asn
            100                 105                 110

Glu Leu Pro Tyr Thr Phe Gly Gln Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230